(19) Europäisches Patentamt
European Patent Office
Office européen des brevets

(11) EP 2 461 792 B1

(12) **EUROPÄISCHE PATENTSCHRIFT**

(45) Veröffentlichungstag und Bekanntmachung des Hinweises auf die Patenterteilung:
**31.08.2016 Patentblatt 2016/35**

(21) Anmeldenummer: **10737328.4**

(22) Anmeldetag: **29.07.2010**

(51) Int Cl.:
***A61Q 5/06*** *(2006.01)* ***A61K 8/86*** *(2006.01)*

(86) Internationale Anmeldenummer:
**PCT/EP2010/061046**

(87) Internationale Veröffentlichungsnummer:
**WO 2011/015514 (10.02.2011 Gazette 2011/06)**

(54) **HAARBEHANDLUNGSMITTEL MIT POLYETHER-MODIFIZIERTEN ORGANISCHEN VERBINDUNGEN UND HAARFESTIGENDEN POLYMEREN**

HAIR TREATMENT AGENTS COMPRISING POLYETHER-MODIFIED ORGANIC COMPOUNDS AND HAIR STYLING POLYMERS

AGENTS DE TRAITEMENT CAPILLAIRE RENFERMANT DES COMPOSÉS ORGANIQUES MODIFIÉS PAR DES POLYÉTHERS ET DES POLYMÈRES FIXATIFS CAPILLAIRES

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **04.08.2009 DE 102009028209**

(43) Veröffentlichungstag der Anmeldung:
**13.06.2012 Patentblatt 2012/24**

(73) Patentinhaber: **Henkel AG & Co. KGaA**
**40589 Düsseldorf (DE)**

(72) Erfinder: **SCHWEINSBERG, Matthias**
**22769 Hamburg (DE)**

(56) Entgegenhaltungen:
**EP-A2- 1 226 813** **WO-A1-2007/096056**
**WO-A2-2009/024450**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Haarbehandlungsmittel, welche neben mindestens einem haarfestigenden Polymer zusätzlich spezielle Polyether-modifizierte organische Verbindungen enthalten sowie die Verwendung dieses Haarbehandlungsmittels zur temporären Frisurgestaltung und ein entsprechendes Haarbehandlungsverfahren.

Haarbehandlungsmittel, die einer permanenten oder temporären Formgebung der Haare dienen, spielen in der Kosmetik eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Oft geht ein hoher Haltegrad unerwünschterweise mit einer hohen Sprödigkeit der Frisur einher. Die mit dem entsprechenden Stylingmittel behandelten Haare sind starr, spröde und wirken unnatürlich fest. Dadurch fühlen sie sich rau und ungepflegt an. Zudem ist der Polymerfilm, den die Mittel bei der Anwendung auf dem Haar hinterlassen in den genannten Fällen so unflexibel, daß er bei Beanspruchung bricht. Hierdurch kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Ein weiteres Problem besteht darin, daß die Produktkonsistenz solcher Produkte vom Verbraucher negativ beurteilt wird, indem diese Produkte als zäh, klebrig und schlecht applizierbar angesehen werden.

Aufgabe der vorliegenden Erfindung war es daher, ein Mittel zur temporären Verformung keratinischer Fasern zur Verfügung zu stellen, das sich durch einen sehr hohen Haltegrad auszeichnet ohne daß dabei auf Flexibilität und ein gepflegtes Haargefühl sowie angenehme Produkthaptik verzichtet werden müsste.

In der Druckschrift WO-A2-2009/024450 werden sternförmige Polyether-haltige Verbindungen beschrieben, die in Mitteln zur Reinigung von Oberflächen Anwendung finden und die Wiederanschmutzung des gereinigten Substrats eindämmen. Die Druckschrift WO-A1-2007096056 beschreibt sternförmige Polyether-haltige Verbindungen sowie deren Einsatz in Oberflächenbehandlungsmitteln, einschließlich Mitteln zur Haarbehandlung. Die europäische Patentanmeldung EP 1 226 813 A2 beschreibt Mittel zur temporären Haarverformung.

Es wurde nunmehr überraschenderweise gefunden, daß sich Stylingprodukte mit einem hohen Haltegrad und einem angenehmem Pflegegefühl im Haar bereitstellen lassen, indem eine Kombination spezieller Inhaltstoffe in die Mittel inkorporiert wird.

Ein erster Gegenstand der Erfindung ist daher ein kosmetisches Mittel, enthaltend in einem kosmetisch akzeptablen Träger

(a) Polyether modifizierte, organische Verbindungen, welche mindestens drei Polyether-Substituenden aufweisen, wobei die Polyether eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette,

und

(b) mindestens ein nichtionisches filmbildendes und/oder nichtionisches festigendes Polymer.

Unter "Polyether-Substituent" wird ein chemisches Strukturfragment verstanden, das eine Polyoxyalkylenkette

aus Ethylenoxideinheiten oder aus Ethylenoxid- und Propylenoxid-Einheiten umfasst, mit einem maximalen Anteil von 50 Gew.-% an Propylenoxid-Einheiten, bezogen auf das Gewicht der Polyoxyalkylenkette, enthält die direkt oder über eine chemische Bindung vermittelndes Strukturfragment an eine organische Verbindung kovalent gebunden ist.

Unter einer Ethylenoxid-Einheit ist im Sinne der vorliegenden Erfindung eine Einheit der allgemeinen Formel (1)

$$*\text{-}CH_2\text{-}CH_2\text{-}O\text{-}* \quad \text{Formel} \quad (1)$$

zu verstehen, und unter einer Propylenoxid-Einheit ist im Sinne der vorliegenden Erfindung eine Einheit der allgemeinen Formel (2)

$$*\text{-}CH_2\text{-}CH(CH_3)\text{-}O\text{-}* \quad \text{Formel} \quad (2)$$

zu verstehen.

Gemäß obiger Formeln (1) und (2) und allen folgenden Formeln steht eine chemische Bindung, die mit dem Symbol * gekennzeichnet ist, für eine freie Valenz des entsprechenden Strukturfragments.

Unter "organische Verbindungen" werden chemische Verbindungen basierend auf einem Kohlenwasserstoff-Strukturfragment verstanden. Entsprechende Kohlenwasserstoffstrukturfragmente leiten sich von linearen, verzweigten, zyklischen oder heterozyklischen Kohlenwasserstoffen ab, wobei all diese jeweils gesättigt, ungesättigt oder aromatisch sein dürfen.

Unter "Polyether modifizierte organische Verbindungen" werden erfindungsgemäß organische Verbindungen verstanden, die mit Polyether-haltigen Substituenden modifiziert sind, wobei die Polyether Substituenden jeweils eine chemische Bindung zu der zu modifizierenden organischen Verbindung ausbilden.

Enthält die Polyoxyalkylenkette des Polyether-Substituenten Ethylenoxid- und Propylenoxid-Einheiten so beträgt der maximale Anteil an Propylenoxid-Einheiten vorzugsweise 40 Gew.-% und besonders bevorzugt maximal 30 Gew.-%, bezogen auf das Gewicht von A.

Die Polyether modifizierten organische Verbindungen sind in den erfindungsgemäßen Mitteln bevorzugt in einer Menge von 0,01 bis 10,0 Gew.-%, besonders bevorzugt von 0,1 bis 2,0 Gew.-%, ganz besonders bevorzugt 0,2 bis 1,0 Gew.-%, jeweils bezogen auf das Gewicht des gesamten Mittels, enthalten.

Besagte Polyether modifizierte, organische Verbindungen, welche mindestens drei Polyether-Substituenden aufweisen werden bevorzugt ausgewählt aus mindestens einer Verbindung der allgemeinen Formel (PE-1)

$$[Q]\text{-}(K'\text{-}A\text{-}K\text{-}T)_n \quad (PE\text{-}1)$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

$$\text{Rest } \text{-}Si(OR)_x(R')_{3\text{-}x},$$

worin R und R' unabhängig voneinander für eine ($C_1$ bis $C_4$)-Alkylgruppe (insbesondere für Methyl oder Ethyl) stehen, x für 1, 2 oder 3 steht,

Q ein organisches Strukturfragment, abgeleitet von linearen, verzweigten, zyklischen oder heterozyklischen Kohlenwasserstoffen, wobei all diese jeweils gesättigt, ungesättigt oder aromatisch sein dürfen,

n steht für eine ganze Zahl von 3 bis 64 (insbesondere für 3, 4, 5, 6, 7 oder 8).

A steht gemäß Formel (PE-1) bevorzugt für ein Strukturfragment der Formel (A1)

$$*\text{-}(OCH_2CH_2)_p\text{-}(OCH_2CH(CH_3))_m\text{-}* \quad (A1)$$

worin

p eine ganze Zahl von 1 bis 500 bedeutet,

m eine ganze Zahl von 0 bis 500 bedeutet und

das Strukturfragment der Formel (A1) einen maximalen Anteil von 50 Gew.-% an Propylenoxid-Einheiten, bezogen auf das Gewicht des Strukturfragments (A1) hat. Die Ethylenoxid- und Propylenoxid-Einheiten gemäß Formel (PE-1) bzw. gemäß Formel (A1) können statistisch verteilt sein oder gradientenartig verteilt sein oder in mindestens zwei Blöcken vorliegen.

Steht die Gruppe A der Verbindungen nach Formel (I) (bzw. nach allen nachfolgenden Formeln enthaltend A) für eine Polyoxyalkylenkette aus Ethylenoxid- und Propylenoxid-Einheiten so beträgt der maximale Anteil an Propylenoxid-Einheiten vorzugsweise 40 Gew.-% und besonders bevorzugt maximal 30 Gew.-%, bezogen auf das Gewicht von A.

Die Reste K beziehungsweise K' gemäß Formel (PE-1) stehen bevorzugt unabhängig voneinander für eine kovalente Bindung, eine Oxygruppe, eine ($C_1$ bis $C_6$)-Alkylengruppe, eine Iminogruppe oder mindestens eine der folgenden Konnektivitäten (K1) bis (K10)

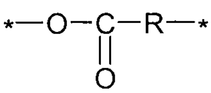 (K1) 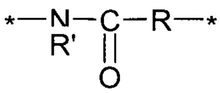 (K2) 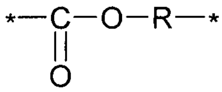 (K3)

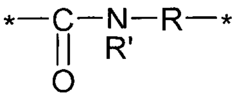 (K4) 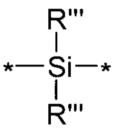 (K5) 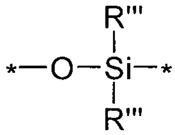 (K6)

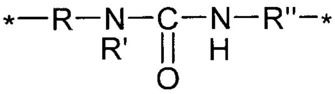 (K7) 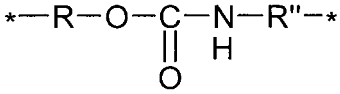 (K8)

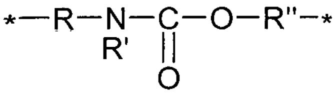 (K9) 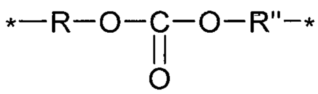 (K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,
R' ein Wasserstoffatom oder eine ($C_1$ bis $C_4$)-Alkylgruppe bedeutet,
R''' unabhängig voneinander eine ($C_1$ bis $C_4$)-Alkylgruppe oder eine Arylgruppe bedeutet.

T steht gemäß Formel (PE-1) bevorzugt

- für ein anionisches Strukturfragment

oder

- für einen Rest $-Si(OR)_x(R')_{3-x}$, worin R und R' unabhängig voneinander für eine ($C_1$ bis $C_4$)-Alkylgruppe (insbesondere für Methyl oder Ethyl) stehen und x für 2 oder 3 steht (bevorzugt für Triethoxysilyl).

Ganz besonders bevorzugt steht das Strukturfragment -K-T gemäß Formeln (PE-1a) bis (PE-1f) für eine Gruppe

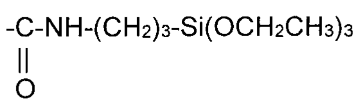

Q steht bevorzugt für einen entsprechenden organischen Rest mit 2 bis 30 Kohlenstoffatomen, besonders bevorzugt mit 2 bis 20 Kohlenstoffatomen.

Q wird bevorzugt abgeleitet von Glyzerin, Monosaccharid, Disaccharid. Ganz besonders bevorzugt leitet sich Q von einer Verbindung ab, ausgewählt aus Sorbitol, 1,5-Anhydro-Sorbitol, 1,4-Anhydrosorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Polyglycerin-3, Glucose, Fructose, Galactose, Ribose, Xylose, Mannose, Saccharose, Cellobiose, Gentiobiose, Isomaltose, Lactose, Lactulose, Maltose, Maltutose, Melibiose, Trehalose, Nigerose, Palatinose, Rutinose, Arabinose.

Ganz besonders bevorzugte Polyether modifizierte organische Verbindungen werden ausgewählt aus mindestens einer Verbindung der Formel (PE-1a) oder (PE-1b) oder (PE-1c) oder (PE-1d) oder (PE-1e) oder (PE-1f) oder Mischungen daraus,

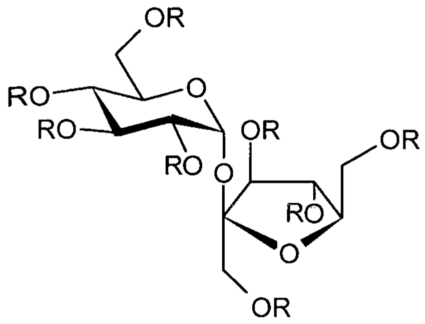

(PE-1a)

worin mindestens drei Gruppen R für eine Gruppe $-(CH_2CH_2O)_p-(CHCH_3CH_2O)_m-K-T$ stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

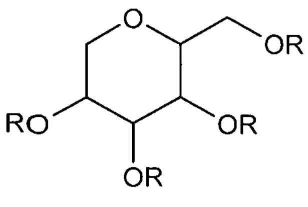

(PE-1b)

worin

mindestens drei Gruppen R für eine Gruppe $-(CH_2CH_2O)_p-(CHCH_3CH_2O)_m-K-T$ stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit

zwei freien Valenzen,

T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

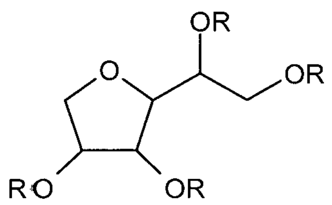

(PE-1c)

worin

mindestens drei Gruppen R für eine Gruppe $-(CH_2CH_2O)_p-(CHCH_3CH_2O)_m$-K-T stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

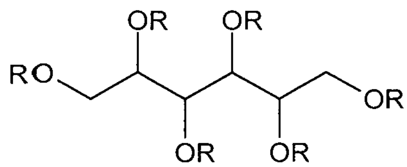

(PE-1d)

worin

mindestens drei Gruppen R für eine Gruppe $-(CH_2CH_2O)_p-(CHCH_3CH_2O)_m$-K-T stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

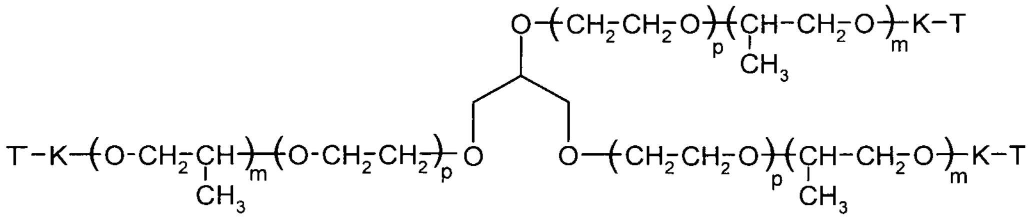

(PE-1f)

worin unabhängig voneinander
p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, hat;

K stehen unabhängig voneinander für eine Konnektivität, ausgewählt aus einer kovalenten Bindung oder aus einem Molekülfragment mit zwei freien Valenzen,

T stehen unabhängig voneinander für ein Molekülfragment umfassend mindestens einen Substituenten ausgewählt aus
anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht.

Die Ethylenoxid- und Propylenoxid-Einheiten können statistisch verteilt sein oder gradientenartig verteilt sein oder in mindestens zwei Blöcken vorliegen.

Es gelten für die Formeln (PE-1a) bis (PE-1f) die zuvor bevorzuten Gruppen K und T. Ganz besonders bevorzugt steht das Strukturfragment -K-T gemäß Formeln (PE-1a) bis (PE-1f) für eine Gruppe

$-C-NH-(CH_2)_3-Si(OCH_2CH_3)_3$
||
O

Es werden erfindungsgemäß solche kosmetischen Mittel bevorzugt, enthaltend in einem kosmetisch akzeptablen Träger

(a) Polyether-modifizierte organische Verbindungen, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit
(ii) mindestens 3 Moläquivalenten mindestens eines Polyethers der Formel (I) erhalten werden

T-K-A-K'-Y (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung

oder aus einem Molekülfragment mit zwei freien Valenzen,

T für ein Molekülfragment steht, umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht

Y für eine gegenüber eine Hydroxygruppe oder Aminogruppe reaktive Gruppe steht,

und

(b) mindestens ein filmbildendes und/oder festigendes Polymer.

Die Angabe der Moläquivalente bezieht sich auf die Stoffmenge der eingesetzten organischen Verbindung.
Für die Reste A, K, K' und T gelten die unter Formel (PE-1) genannten, bevorzugten Ausführungsformen.
Bevorzugt steht der Rest Y gemäß Formel (I) für

- eine Isocyanatfunktion, (die Isocyanatfunktion ist besonders bevorzugt)
- eine Gruppe $*-Si(OR)_x(R')_{3-x}$ worin R und R' unabhängig voneinander für eine ($C_1$ bis $C_4$)-Alkylgruppe (insbesondere für Methyl oder Ethyl) stehen und x für 1, 2 oder 3 steht,
- eine Gruppe $*-Si(R)_x(R')_{3-x}$ worin R für ein Halogenatom steht (bevorzugt für Chlor oder Brom) und R' unabhängig voneinander für eine ($C_1$ bis $C_4$)-Alkylgruppe (insbesondere für Methyl oder Ethyl) stehen und x für 1, 2 oder 3 steht,
- eine Gruppe T-K-C(O)-O-C(O)-*, worin T und K gemäß Formel (I) definiert sind,
- eine Gruppe *-C(O)-Hal, worin Hal für Chlor oder Brom steht,
- eine Epoxygruppe,
- eine Formylgruppe.

Die Polyether-modifizierten organischen Verbindungen dieser Ausführungsform sind in den erfindungsgemäßen Mitteln bevorzugt in einer Menge von 0,01 bis 10,0 Gew.-%, besonders bevorzugt von 0,1 bis 2,0 Gew.-%, ganz besonders bevorzugt 0,2 bis 1,0 Gew.-%, jeweils bezogen auf das Gewicht des gesamten Mittels, enthalten.
Die Polyether der Formel (I) besitzen bevorzugt eine Molmasse von 1 bis 200 kDa, besonders bevorzugt von 1 bis 10 kDa.
Die im Rahmen der Erfindung zur Herstellung der Polyether modifizierten organischen Verbindungen der Komponente (a) einsetzbaren Polyether sind erhältlich durch Umsetzung von mindestens einer Verbindung der Formel (II):

X-A-X' (II)

worin

A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,
X und X' unabhängig voneinander für ein Strukturfragment stehen, enthaltend eine Gruppe OH, $NH_2$ oder NHR

mit mindestens 2 Äquivalenten einer Verbindung der Formel (III)

Y-K-T (III)

worin,

Y für eine gegenüber -OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht.

Die Angabe der Moläquivalente bezieht sich auf die Stoffmenge der eingesetzten Verbindung(en) der Formel (II).

Gemäß obigem Herstellverfahren zur Herstellung der Verbindungen der Formel (I) werden als Verbindungen der Formel (II) beispielsweise dihydroxyterminierte Polyoxyalkylen-Diole, diaminoterminierte Polyoxyalkylen-Diamine, monohydroxy-monoamin-terminierte Polyoxyalkylen-Monol-Monoamine, monohydroxy-monoalkoxy-terminierte Polyoxyalkylen-Monole oder monoamino-monoalkoxy-terminierte Polyoxyalkylen-Monoamine eingesetzt, worunter die Diamine und Diole bevozugt sind.

Vorzugsweise stehen die Reste X und X' der Formel (II) unabhängig voneinander für OH, $NH_2$ und NHR, besonders bevorzugt für OH und $NH_2$.
Bevorzugt steht der Rest R in den Gruppen NHR und $NR_2$ der Formel (III) für eine lineare oder verzweigte Alkylgruppen mit 1 bis 10, vorzugsweise 1 bis 6 Kohlenstoffatomen.

Das zahlenmittlere Molekulargewicht der Verbindung der Formel (II) beträgt vorzugsweise 100 bis 50000 g/mol, besonders bevorzugt 500 bis 30000 g/mol, ganz besonders bevorzugt 1000 bis 20000, besser noch 2000 bis 18000 g/mol, und lässt sich wie im Beispielteil durch Endgruppenbestimmung ermitteln.

Für die Reste A, K, K' und T gelten die unter Formel (PE-1) genannten, bevorzugten Ausführungsformen.

Es werden erfindungsgemäß außerdem solche kosmetischen Mittel bevorzugt, enthaltend in einem kosmetisch akzeptablen Träger

(a) Polyether-modifizierte organische Verbindungen, welche durch Umsetzung von

(i) organischen Polyether-Polyol-Verbindungen umfassend

- mindestens drei Polyoxyalkylenketten aus Ethylenoxideinheiten oder aus Ethylenoxid- und Propylenoxid-Einheiten, mit einem maximalen Anteil von 50 Gew.-% an Propylenoxid-Einheiten, bezogen auf das Gewicht der Polyoxyalkylenkette,
- mindestens drei Hydroxygruppen

mit
(ii) mindestens 3 Moläquivalenten mindestens einer Verbindung der Formel (III)

Y-K-T (III)

worin,

Y für eine gegenüber -OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht.

und

(b) mindestens ein filmbildendes und/oder festigendes Polymer.

Entsprechende im Schritt (i) erfindungsgemäß einsetzbare Polyether-Polyole sind beispielsweise VORANOL®, TERRALOX®, SYNALOX® und DOWFAX® der Dow-Chemical Corporation, SORBETH® der Glyco-Chemicals Inc., GLUCAM® der Amerchol Corp., oder Lupranol® und Pluronic® der BASF.

Bevorzugt werden im Rahmen dieser Ausführungsform als Verbindung der Formel (III) mindestens eine Verbindung der allgemeinen Formel (III-1) eingesetzt

$$Y\text{-}K\text{-}Si(OR)_xR'_{3-x} \qquad (III\text{-}1)$$

worin,

Y für eine gegenüber OH, $NH_2$, NHR und/oder $NR_2$ reaktive Gruppe (insbesondere für eine Isocyanatgruppe, ein Halogenatom, eine Carbonsäureanhydridgruppe, eine Halogencarbonylgruppe (insbesondere Chlorcarbonyl), eine Epoxygruppe, eine Formylgruppe), steht,

K wie unter Formel (I) (bzw. wie die vorgenannten bevorzugten Konnektivitäten) definiert ist,
R für eine ($C_1$ bis $C_4$)-Alkylgruppe oder eine ($C_2$ bis $C_4$)-Acylgruppe (insbesondere für Ethyl oder Methyl) steht,
R' für eine ($C_1$ bis $C_4$)-Alkylgruppe (insbesondere Methyl oder Ethyl) steht,
x für 1, 2 oder 3 steht.

Zu den Verbindungen der allgemeinen Formel (III-1) gehören alle funktionellen Silan-Derivate, die zur Reaktion gegenüber Y-Gruppen der Formel (III-1) befähigt sind. Beispiele sind Acrylat-Silane wie (3-Acryloxypropyl)trimethoxysilan, (Acryloxymethyl)triethoxysilan und (Acryloxymethyl)methyl-dimethoxysilan, Isocyanato-Silane wie (3-Isocyanatopropyl)trimethoxysilan, (3-Isocyanatopropyl)triethoxysilan, (Isocyanatomethyl)methyl-Dimethoxysilan und (Isocyanatomethyl)trimethoxysilan, Aldehyde-Silane wie Triethoxysilylundecanal und Triethoxysilylbutyraldehyde, Epoxy-Silane wie (3-Glycidoxypropyl)trimethoxysilan, Anhydridsilane wie 3-(Triethoxysilyl)propylbernsteinsäureanhydrid, Halogen-Silane wie Chloromethyltrimethoxysilan, 3-Chloropropylmethyldimethoxysilan, sowie Tetraethylsilikat (TEOS), die kommerziell beispielsweise bei der Wacker Chemie GmbH (Burghausen), der Gelest, Inc. (Morrisville, USA) oder ABCR GmbH & Co. KG (Karlsruhe) erhältlich sind oder nach bekannten Verfahren hergestellt werden können. Besonders bevorzugt werden Isocyanato-Silane bzw. Anhydrid-Silane. Bei einer vollständigen Umsetzung aller Hydroxyenden mit Isocyanatosilanen erhält man komplett silylierte Polyether. Die Gruppe K enthält in einem solchen Fall nur die Atomgruppe, die im Ausgangs-Isocyanatosilan zwischen der Isocyanatogruppe und der Silylgruppe steht. Bei einer vollständigen Umsetzung aller Hydroxyenden mit Anhydrid-Silanen, beispielsweise 3-(Triethoxysilyl)propylbernsteinsäureanhydrid, erhält man ebenfalls komplett silylierte Polyether. Die Gruppe K' enthält in einem solchen Fall nur die Atomgruppe, die im Ausgangs-Anhydridsilan zwischen der Anhydridgruppe und der Silylgruppe steht.

Stehen die Reste X und X' in der allgemeinen Formel (II) für OH, $NH_2$ oder NHR, so erfolgt die Reaktion mit den Verbindungen der allgemeinen Formel (III) bzw. (III-1) üblicherweise entweder unter Abspaltung der Verbindung HY - wie beispielsweise im Falle der Umsetzung einer OH-Gruppe mit einem Monohalogensilan (K = direkte Bindung) - oder aber unter Addition - wie beispielsweise im Falle der Umsetzung einer OH-Gruppe mit einem Isocyanatoalkylsilan (Bildung eines Urethans).

Vorzugsweise stehen die Reste X und X' der Formel (II) unabhängig voneinander für OH, $NH_2$ und NHR, besonders bevorzugt für OH und $NH_2$.

Bevorzugt steht der Rest R in den Gruppen NHR, $NR_2$ und OR der Formel (III-1) für eine lineare oder verzweigte Alkylgruppen mit 1 bis 10, vorzugsweise 1 bis 6 Kohlenstoffatomen.

Bei der Reaktion zwischen den Verbindungen der Formel (I) und den Verbindungen der Formel (II) wird mindestens ein Wasserstoffatom, vorzugsweise werden bis zu vier Wasserstoffatome der OH- und/oder $NH_2$-Gruppen mit je einem Molekül der Verbindung der allgemeinen Formel (II) umgesetzt, so dass zumindest monosilylierte, im Falle der Diaminoverbindungen der allgemeinen Formel (I) bis zu vierfach silylierte Polyether entstehen.

Ganz besonders bevorzugte Polyether zur Herstellung der erfindungsgemäßen Polyether-modifizierten orgnaischen Verbindungen werden ausgewählt aus mindestens einer Verbindung der Formeln (I-1a) oder (I-1b)

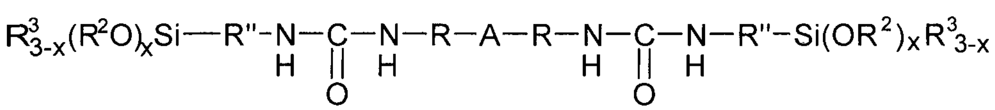

(I-1a)

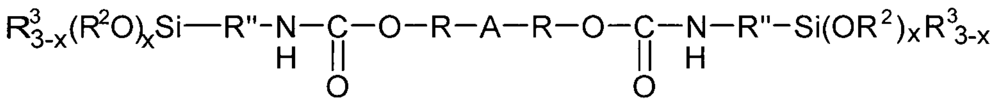

(I-1b)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl, Phenylen,

$R^1$ für eine ($C_1$ bis $C_6$)-Alkylgruppe, ein Wasserstoffatom oder eine Gruppe $R^3{}_{3-x}(R^2O)_xSi$-K-steht,

$R^2$ für eine ($C_1$ bis $C_4$)-Alkylgruppe (insbesondere für Methyl oder Ethyl) steht,

$R^3$ für eine ($C_1$ bis $C_6$)-Alkylgruppe oder eine Arylgruppe (insbesondere Methyl) steht,

x für 1, 2, 3 (insbesondere 3) steht.

Im Rahmen einer weiteren Ausführungsform (zur Herstellung) der erfindungsgemäßen Polyether-modifizierten organischen Verbindungen umfasst mindestens ein Molekülfragment T der Formel (PE-1), bzw. Formel (I) bzw. Formel (III) mindestens eine anionische Gruppe.

Bei den Molekülfragmenten T der Formel (I) bzw. Formel (III) mit mindestens einer anionischen Gruppe handelt es sich erfindungsgemäß vorzugsweise um ein Molekülfragment, das 1 bis 5, vorzugsweise 3, 4 oder 5, deprotonierbare Säuregruppen umfasst. Die anionischen Gruppen bzw. die deprotonierbaren Säuregruppen der besagten Molekülfragmente T der Formel (I) bzw. Formel (III) werden bevorzugt ausgewählt aus Carboxylgruppe und/oder Sulfonsäuregruppe und/oder Phosphat bzw. deren jeweiligen Salzformen (insbesondere Carboxylgruppe und/oder Sulfonsäuregruppe bzw. deren jeweiligen Salzformen, besonders bevorzugt Carboxylgruppe bzw. deren Salzform).

In einer besonders bevorzugten Ausführungsform enthält das Molekülfragment T der Formel (I) bzw. Formel (III) mindestens eine, vorzugsweise mindestens zwei, besonders bevorzugt 1 bis 5, vor allem 2 bis 5, insbesondere 2, 3, 4 oder 5 Carboxymethyl-Einheiten. In einer ganz besonders bevorzugten Ausführungsform handelt es sich bei dem besagten Molekülfragment um eine Ethylendiamintriacetat-Einheit, die über eines ihrer Stickstoff-Atome kovalent an die Konnektivität K der Formel (I) bzw. Formel (III) gebunden ist.

In einer erfindungsgemäß bevorzugten Ausführungsform zur Herstellung der erfindungsgemäßen Polyether-modifizierten organischen Verbindungen handelt es sich bei dem Molekülfragment T der Formel (PE-1) bzw. der Formel (I) bzw. Formel (III) um eine Silyl-Gruppe der allgemeinen Formel (IV)

$$-CR^a{}_2-Si(OR^b)_r(R^c)_{3-r} \qquad (IV),$$

wobei

$R^a$ für Wasserstoff oder $C_{1-6}$-Alkyl steht,

$R^b$ für $-Si(R^d)_t(R^e)_{3-t}$ steht,

$R^c$ für ($C_1$ bis $C_6$)-Alkyl, ($C_1$ bis $C_6$)-Alkoxy oder Hydroxy, vorzugsweise für ($C_1$ bis $C_6$)-Alkoxy oder Hydroxy, steht,

$R^d$ für eine negativ geladene Gruppe steht,

$R^e$ für ($C_1$ bis $C_6$)-Alkyl, $C_{1-6}$-Alkoxy oder Hydroxy, vorzugsweise für ($C_1$ bis $C_6$)-Alkoxy oder Hydroxy, steht,

r für eine Zahl von 1 bis 3, vorzugsweise für 1, steht,

t für eine Zahl von 1 bis 3, vorzugsweise für 1, steht.

$R^d$ steht vorzugsweise für eine Einheit, die 1-5, vorzugsweise 3, 4 oder 5, Säuregruppen, insbesondere Carbonsäuregruppen, umfasst.

Besonders bevorzugt steht $R^d$ der Formel (IV) für eine Gruppe

$$*-B-[(N(CH_2COOM)-B')_y-N(CH_2COOM)_2]$$

wobei

B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,

B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,

M unabhängig voneinander für ein Wasserstoffatom oder ein Äquivalent eines ein- oder mehrwertigen Kations,

y 1 oder 2 (vorzugsweise 1) bedeutet.

Wenn die obige Gruppe als Säure vorliegt, bedeutet der Rest M ein Wasserstoffatom. Die Fragmente -COOH bilden in diesem Fall eine Carboxylgruppe. Wenn die obige Gruppe in ihrer Salzform vorliegt (Carboxylat), steht M für ein Äquivalent eines ein- oder mehrwertigen Kations. Das ein oder mehrwertige Kation $M^{z+}$ mit einer Ladungszahl z von eins oder höher dient lediglich aus Gründen der Elektroneutralität zur Kompensation der einfach negativen Ladung des bei Salzbildung vorliegenden Carboxylats. Das dafür zu verwendende Äquivalent des entsprechenden Kations beträgt 1/z. Das Fragment -COOM steht im Fall der Salzbildung für die Gruppe: -COO- 1/z ($M^{z+}$)

Als ein- oder mehrwertige Kationen $M^{z+}$ kommen prinzipiell alle Kationen in Frage, die physiologisch verträglich sind. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der obigen Gruppe $R^d$ in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Ein ganz besonders bevorzugtes Molekülfragment T der Formel (PE-1) bzw. Formel (I) bzw. Formel (III) zur Herstellung der erfindungsgemäßen Polyether-modifizierten organischen Verbindungen folgt der allgemeinen Formel

$$*\text{-B-}[(N(CH_2COOM)\text{-B'})_y\text{-}N(CH_2COOM)_2]$$

wobei

B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,

B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,

M unabhängig voneinander für ein Wasserstoffatom oder ein Äquivalent eines ein- oder mehrwertigen Kations,

y 1 oder 2 (vorzugsweise 1) bedeutet.

Für den Rest M der obigen Formel gilt das zuvor Gesagte (*vide supra*).

Besonders bevorzugte Mittel dieser Ausführungsform weisen Polyether-modifizierten organischen Verbindungen auf, die mindestens einen Polyether mit mindestens einem Molekülfragment T der besagten allgemeinen Formel

$*\text{-B-}[(N(CH_2COOM)\text{-B'})_y\text{-}N(CH_2COOM)_2]$ enthalten.

Besonders bevorzugte Molekülfragmente T aus Formel (PE-1) bzw. Formel (I) bzw. Formel (III) mit der Formel $*\text{-B-}[(N(CH_2COOM)\text{-B'})_y\text{-}N(CH_2COOM)_2]$ werden ausgewählt aus mindestens einem Vertreter der Gruppe, die gebildet wird aus 3-N-Carboxylmethyl-N-(2'-N',N'-di(carboxymethylamino)ethyl)-aminopropyl (B=Propan-1,3-diyl, B'=Ethan-1,2-diyl, y=1, M wie oben), 3-N-Carboxylmethyl-N-(2'-N',N'-di(carboxymethylamino)ethyl)-N"-carboxymethylamino-ethyl)aminopropyl (B=Propan-1,3-diyl, B'=Ethan-1,2-diyl, y=2, M wie oben). Dabei ist 3-N-Carboxylmethyl-N-(2'-N',N'-di(carboxymethylamino)-ethyl)aminopropyl ganz besonders bevorzugt.

Ganz besonders bevorzugte Verbindungen zur Herstellung der erfindungsgemäßen Polyether-modifizierten organischen Verbindungen verwendbare Verbindungen werden ausgewählt aus mindestens einer Verbindung der Formel (I-1c)

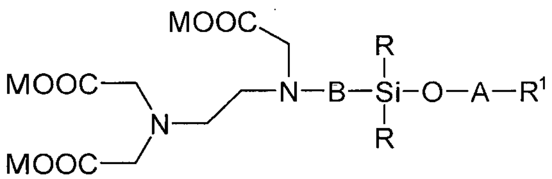

(I-1c)

worin

- A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
- B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,
- M unabhängig voneinander für ein Wasserstoffatom oder ein Äquivalent eines ein- oder mehrwertigen Kations,
- $R^1$ für Gruppe $R^3_{3-x}(R^2O)_xSi$-K- steht worin $R^2$ und $R^3$ unabhängig voneinander stehen für eine ($C_1$ bis $C_4$)-Alkylgruppe (insbesondere Methyl oder Ethyl), x eine ganze Zahl 1, 2 oder 3 (insbesondere 3) bedeutet, K steht für eine Konnektivität, ausgewählt aus einer kovalenten Bindung oder aus einem Molekülfragment mit zwei freien Valenzen,

- R unabhängig voneinander für eine ($C_1$ bis $C_4$)-Alkylgruppe (insbesondere für Methyl oder Ethyl) oder eine ($C_1$ bis $C_6$)-Alkoxygruppe (insbesondere Methoxy oder Ethoxy) steht.

Diese wird als Verbindung der Formel (III) bevorzugt mit Polyether-Polyolen gemäß zuvor beschriebenem Verfahren zu den erfindungsgemäßen Polyether modifizierten organischen Verbindungen umgesetzt.

Für die oben genannte Formeln (I-1c)gelten die bevorzugten Ausführungsformen bezüglich der zuvor genannten bevorzugten Merkmale für A, B, B', M, $R^1$ und R *mutatis mutandis*.

Die Benetzbarkeit der durch die erfindungsgemäßen Mittel erzielten Beschichtungen mit Wasser ist ein empfindliches Maß für deren Hydrophilie oder Hydrophobie. Der Kontaktwinkel eines Wassertropfen auf einem planaren Substrat im umgebenden Medium Luft resultiert aus den Oberflächenenergien der Beschichtung und des Wassers sowie der Grenzflächenenergie zwischen Wasser und der Beschichtung nach der Youngschen Gleichung. Im Fall maximaler Hydrophilie geht der Kontaktwinkel gegen 0°. Im Fall maximaler Hydrophobie geht der Kontaktwinkel gegen 180°. In der Praxis wird häufig der fortschreitende (*advancing*) Kontaktwinkel und der rückziehende (*receding*) Kontaktwinkel gemessen. Im Idealfall sollte der Unterschied zwischen beiden gleich Null sein. Im Realfall existiert ein Unterschied, auch Kontaktwinkelhysterese genannt, der auf Oberflächenrauigkeit, Inhomogenitäten und Verunreinigungen zurückgeführt wird.

Vorzugsweise besitzen die erfindungsgemäßen Beschichtungen einen mit dem Sessile-Drop-Verfahren (zur Durchführung siehe Beispielteil) gemessenen statischen Wasserkontaktwinkel von höchstens 90°, besser höchstens 70°, besonders bevorzugt höchstens 55° und ganz besonders bevorzugt von höchstens 45°. Es werden in vielen Fällen jedoch auch Wasserkontaktwinkel von 40° und weniger erreicht. Ganz besonders bevorzugt enthalten die erfindungsgemäßen Mittel Polyether der Formel (I), so dass nach der Behandlung keratinhaltiger Fasern, insbesondere menschlicher Haare, mit diesem erfindungsgemäßen Mittel die erzielte Beschichtung einen Kontaktwinkel von 20° bis 60° aufweist.

Das erfindungsgemäße Mittel enthält neben den erfindungsgemäßen Polyether-modifizierten organischen Verbindungen weiterhin zwingend mindestens ein nichtionisches filmbildendes und/oder nichtionisches festigendes Polymer. Diese Polymere sind von den besagten Polyether-modifizierten organischen Verbindungen verschieden.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers werden häufig der so genannte curl-retention - Test oder der Dreipunkt-Biegetest angewendet.

Im Sinne der Erfindung bevorzugte Mittel enthalten die filmbildenden und/oder festigenden Polymere in einer Menge von 0,1 Gew.-% bis 20,0 Gew.-%, besonders bevorzugt von 0,2 Gew.-% bis 10,0 Gew.-%, ganz besonders bevorzugt von 0,5 Gew.-% bis 5,0 Gew.-%, jeweils bezogen auf das Gewicht des Mittels.

Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die filmbildenden nichtionischen und/oder festigenden nichtionischen Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten.

Die filmbildenden nichtionischen und/oder festigenden nichtionischen Polymere werden wiederum bevorzugt ausgewählt aus mindestens einem Polymer der Gruppe, die gebildet wird, aus

- Homopolymeren und nichtionischen Copolymeren des N-Vinylpyrrolidons,
- nichtionischen Copolymeren des Isobutens.

Geeignete Polyvinylpyrrolidone sind beispielsweise Handelsprodukte wie Luviskol® K 90 oder Luviskol® K 85 der Firma BASF SE.

Geeignete Polyvinylalkohole werden beispielsweise unter den Handelsbezeichnungen Elvanol® von Du Pont oder Vinol® 523/540 von der Firma Air Products vertrieben.

Geeignetes Polyvinylacetat wird beispielsweise unter dem Handelsnamen Vinac® als Emulsion von der Firma Air Products vertrieben.

Mittel, die als filmbildendes nichtionisches und/oder festigendes nichtionisches Polymer mindestens ein Polymer ausgewählt aus der Gruppe, die gebildet wird aus

- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di($C_1$ bis $C_4$)-Alkylamino-($C_2$ bis $C_4$)-alkylacrylamid,
- Copolymere aus N-Vinylpyrrolidon mit N,N-Di($C_1$ bis $C_4$)-Alkylamino-($C_2$ bis $C_4$)-alkylacrylamid, sind erfindungsgemäß ganz besonders bevorzugt.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche ausgewählt werden aus mindestens einer Verbindung der allgemeinen Formel (PE-1)

$$[Q]\!-\!\left(K'\text{-}A\text{-}K\text{-}T\right)_n \qquad \text{(PE-1)}$$

wobei Q, K', A, K, T und n wie zuvor beschrieben definiert sind.
und
(b) Polyvinylpyrrolidon.

Im Rahmen dieser Ausführungsform eignen sich solche Mittel bevorzugt, enthaltend in einem kosmetisch akzeptablen Träger

(a) Polyether-modifizierte organische Verbindungen, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit
(ii) mindestens 3 Moläquivalenten mindestens eines Polyethers der Formel (I) erhalten werden

$$T\text{-}K\text{-}A\text{-}K'\text{-}Y \qquad \text{(I)}$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung oder aus einem Molekülfragment mit zwei freien Valenzen,

T für ein Molekülfragment steht, umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht

Y für eine gegenüber eine Hydroxygruppe oder Aminogruppe reaktive Gruppe steht,

und

(b) Polyvinylpyrrolidon.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche durch Umsetzung von

(i) organischen Polyether-Polyol-Verbindungen umfassend

- mindestens drei Polyoxyalkylenketten aus Ethylenoxideinheiten oder aus Ethylenoxid- und Propylenoxid-Einheiten, mit einem maximalen Anteil von 50 Gew.-% an Propylenoxid-Einheiten, bezogen auf das Gewicht der Polyoxyalkylenkette,
- mindestens drei Hydroxygruppen

mit
(ii) mindestens 3 Moläquivalenten mindestens einer Verbindung der Formel (III)

Y-K-T (III)

worin,

Y für eine gegenüber-OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht.

und

(b) Polyvinylpyrrolidon.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche ausgewählt werden aus mindestens einer Verbindung der allgemeinen Formel (PE-1)

$$[Q]\text{—}(K'\text{-}A\text{-}K\text{-}T)_n \qquad \text{(PE-1)}$$

wobei Q, K', A, K, T und n wie zuvor beschrieben definiert sind.

und

(b) mindestens Copolymer, welches aus den Monomeren N-Vinylpyrrolidon und Vinylacetat - insbesondere aus keinem weiteren Monomeren - hergestellt wird.

Im Rahmen dieser Ausführungsform eignen sich solche Mittel als ganz besonders bevorzugt, enthaltend in einem kosmetisch akzeptablen Träger

(a) Polyether-modifizierte organische Verbindungen, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit
(ii) mindestens 3 Moläquivalenten mindestens eines Polyethers der Formel (I) erhalten werden

T-K-A-K'-Y (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,

T für ein Molekülfragment steht, umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht

Y für eine gegenüber eine Hydroxygruppe oder Aminogruppe reaktive Gruppe steht,

und

(b) mindestens Copolymer, welches aus den Monomeren N-Vinylpyrrolidon und Vinylacetat - insbesondere aus keinem weiteren Monomeren - hergestellt wird.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche durch Umsetzung von

(i) organischen Polyether-Polyol-Verbindungen umfassend

- mindestens drei Polyoxyalkylenketten aus Ethylenoxideinheiten oder aus Ethylenoxid- und Propylenoxid-Einheiten, mit einem maximalen Anteil von 50 Gew.-% an Propylenoxid-Einheiten, bezogen auf das Gewicht der Polyoxyalkylenkette,
- mindestens drei Hydroxygruppen

mit
(ii) mindestens 3 Moläquivalenten mindestens einer Verbindung der Formel (III)

$$Y\text{-}K\text{-}T \qquad \text{(III)}$$

worin,

Y für eine gegenüber -OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht.

und

(b) mindestens Copolymer, welches aus den Monomeren N-Vinylpyrrolidon und Vinylacetat

- insbesondere aus keinem weiteren Monomeren - hergestellt wird.

Dabei ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt.
Geeignete Copolymerisate aus Vinylpyrrolidon und Vinylacetat sind beispielsweise unter dem Warenzeichen Luviskol® VA 37, Luviskol® VA 55, Luviskol® VA 64 und Luviskol® VA 73 von der Firme BASF SE erhältlich.
Weitere bevorzugte erfindungsgemäße Mittel sind dadurch gekennzeichnet, daß sie als nichtionisches filmbildendes und/oder nichtionisches festigendes Polymer mindestens ein Copolymer (n1) enthalten, das mindestens eine Struktureinheit gemäß Formel (M-I), mindestens eine weitere Struktureinheit gemäß Formel (M-VII) und mindestens eine weitere Struktureinheit gemäß Formel (M-VIII) enthält

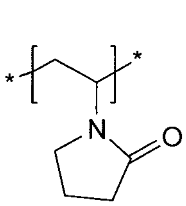 (M-I) 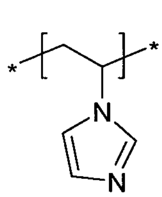 (M-VII) 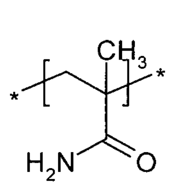 (M-VIII).

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (n1) ausschließlich aus Struktureinheiten der Formel (M1-a), (I), (VII) und (VIII) aufgebaut und lassen sich durch die allgemeine Formel (Poly4)

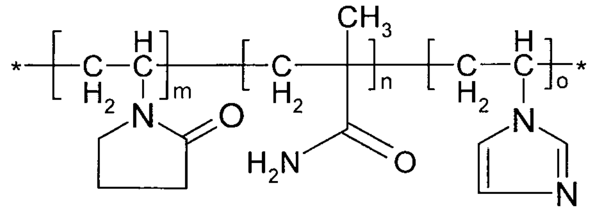
(Poly4)

beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche ausgewählt werden aus mindestens einer Verbindung der allgemeinen Formel (PE-1)

$$[Q]\!-\!(K'\text{-}A\text{-}K\text{-}T)_n \qquad \text{(PE-1)}$$

wobei Q, K', A, K, T und n wie zuvor beschrieben definiert sind.

und

(b) mindestens ein nichtionisches filmbildendes und/oder nichtionisches festigendes Polymer, umfassend mindestens eine Struktureinheit gemäß Formel (M-I), mindestens eine weitere Struktureinheit gemäß Formel (M-VII) und mindestens eine weitere Struktureinheit gemäß Formel (M-VIII)

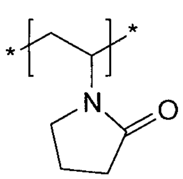
(M-I)

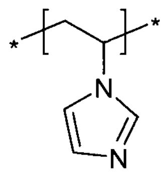
(M-VII)

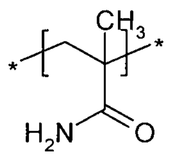
(M-VIII).

Im Rahmen dieser Ausführungsform eignen sich solche Mittel als ganz besonders bevorzugt, enthaltend in einem kosmetisch akzeptablen Träger

(a) Polyether-modifizierte organische Verbindungen, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit
(ii) mindestens 3 Moläquivalenten mindestens eines Polyethers der Formel (I) erhalten werden

T-K-A-K'-Y (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung oder aus einem Molekülfragment mit zwei freien Valenzen,

T für ein Molekülfragment steht, umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht

Y für eine gegenüber eine Hydroxygruppe oder Aminogruppe reaktive Gruppe steht,

und
(b) mindestens ein nichtionisches filmbildendes und/oder nichtionisches festigendes Polymer, umfassend mindestens eine Struktureinheit gemäß Formel (M-I), mindestens eine weitere Struktureinheit gemäß Formel (M-VII) und mindestens eine weitere Struktureinheit gemäß Formel (M-VIII)

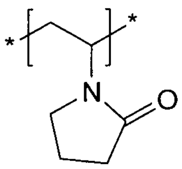 (M-I) 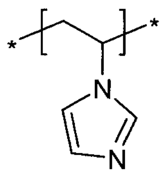 (M-VII) 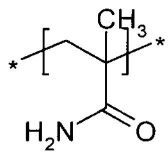 (M-VIII).

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche durch Umsetzung von

(i) organischen Polyether-Polyol-Verbindungen umfassend

- mindestens drei Polyoxyalkylenketten aus Ethylenoxideinheiten oder aus Ethylenoxid- und Propylenoxid-Einheiten, mit einem maximalen Anteil von 50 Gew.-% an Propylenoxid-Einheiten, bezogen auf das Gewicht der Polyoxyalkylenkette,
- mindestens drei Hydroxygruppen

mit
(ii) mindestens 3 Moläquivalenten mindestens einer Verbindung der Formel (III)

Y-K-T (III)

worin,

Y für eine gegenüber $-OH$, $-NH_2$, $-NHR$, $-NR_2$ reaktive Gruppe steht,

K steht für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,

x für 1, 2 oder 3 steht.

und

(b) mindestens ein nichtionisches filmbildendes und/oder nichtionisches festigendes Polymer, umfassend mindestens eine Struktureinheit gemäß Formel (M-I), mindestens eine weitere Struktureinheit gemäß Formel (M-VII) und mindestens eine weitere Struktureinheit gemäß Formel (M-VIII) enthält

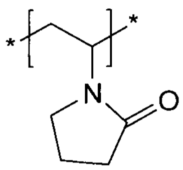 (M-I) 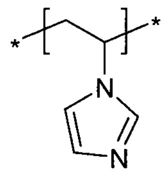 (M-VII) 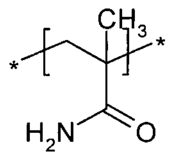 (M-VIII).

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Weiterhin hat es sich als bevorzugt gezeigt, zur Lösung der Aufgabe Mittel zu verwenden, die mindestens ein nichtionisches filmbildendes und/oder nichtionisches festigendes Polymer enthalten, umfassend mindestens eine Struktureinheit der Formel (M-I) und mindestens eine Struktureinheit der Formel (M-III)

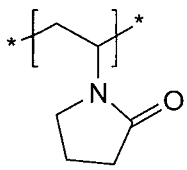 (M-I) 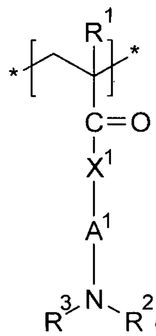 (M-III)

worin

$R^1$ für ein Wasserstoffatom oder eine Methylgruppe steht,
$X^1$ für ein Sauerstoffatom oder eine Gruppe NH steht,
$A^1$ für eine Gruppe Ethan-1,2-diyl, Propan-1,3-diyl oder Butan-1,4-diyl steht
$R^2$ und $R^3$ stehen unabhängig voneinander für eine ($C_1$ bis $C_4$)-Alkylgruppe.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche ausgewählt werden aus mindestens einer Verbindung der allgemeinen Formel (PE-1)

$$[Q]-(K'\text{-}A\text{-}K\text{-}T)_n \qquad \text{(PE-1)}$$

wobei Q, K', A, K, T und n wie zuvor beschrieben definiert sind.
und
(b) mindestens ein nichtionisches filmbildendes und/oder nichtionisches festigendes Polymer, umfassend mindestens eine Struktureinheit der Formel (M-I) und mindestens eine Struktureinheit der Formel (M-III)

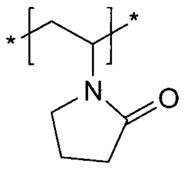 (M-I)

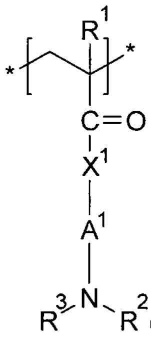 (M-III)

worin

$R^1$ für ein Wasserstoffatom oder eine Methylgruppe steht,
$X^1$ für ein Sauerstoffatom oder eine Gruppe NH steht,
$A^1$ für eine Gruppe Ethan-1,2-diyl, Propan-1,3-diyl oder Butan-1,4-diyl steht
$R^2$ und $R^3$ stehen unabhängig voneinander für eine ($C_1$ bis $C_4$)-Alkylgruppe.

Im Rahmen dieser Ausführungsform eignen sich solche Mittel als ganz besonders bevorzugt, enthaltend in einem kosmetisch akzeptablen Träger

(a) Polyether-modifizierte organische Verbindungen, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit
(ii) mindestens 3 Moläquivalenten mindestens eines Polyethers der Formel (I) erhalten werden

T-K-A-K'-Y (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T für ein Molekülfragment steht, umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht

Y für eine gegenüber eine Hydroxygruppe oder Aminogruppe reaktive Gruppe steht,

und

(b) mindestens ein nichtionisches filmbildendes und/oder nichtionisches festigendes Polymer, umfassend mindestens eine Struktureinheit der Formel (M-I) und mindestens eine Struktureinheit der Formel (M-III)

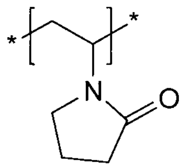 (M-I)

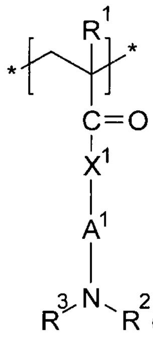 (M-III)

worin

$R^1$ für ein Wasserstoffatom oder eine Methylgruppe steht,
$X^1$ für ein Sauerstoffatom oder eine Gruppe NH steht,
$A^1$ für eine Gruppe Ethan-1,2-diyl, Propan-1,3-diyl oder Butan-1,4-diyl steht
$R^2$ und $R^3$ stehen unabhängig voneinander für eine ($C_1$ bis $C_4$)-Alkylgruppe.

Es gelten weiterhin insbesondere solche Mittel im Rahmen dieser Ausführungsform als ganz besonders bevorzugt, die in einem kosmetisch akzeptablen Träger enthalten

(a) Polyether-modifizierte organische Verbindungen, welche durch Umsetzung von

(i) organischen Polyether-Polyol-Verbindungen umfassend

- mindestens drei Polyoxyalkylenketten aus Ethylenoxideinheiten oder aus Ethylenoxid- und Propylenoxid-Einheiten, mit einem maximalen Anteil von 50 Gew.-% an Propylenoxid-Einheiten, bezogen auf das Gewicht der Polyoxyalkylenkette,
- mindestens drei Hydroxygruppen

mit
(ii) mindestens 3 Moläquivalenten mindestens einer Verbindung der Formel (III)

Y-K-T (III)

worin,

Y für eine gegenüber -OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,

T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht.

und

(b) mindestens ein nichtionisches filmbildendes und/oder nichtionisches festigendes Polymer, umfassend mindestens eine Struktureinheit der Formel (M-I) und mindestens eine Struktureinheit der Formel (M-III)

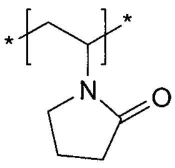 (M-I)

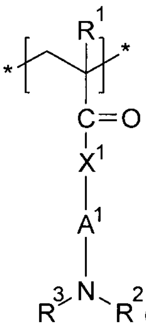 (M-III)

worin

$R^1$ für ein Wasserstoffatom oder eine Methylgruppe steht,
$X^1$ für ein Sauerstoffatom oder eine Gruppe NH steht,
$A^1$ für eine Gruppe Ethan-1,2-diyl, Propan-1,3-diyl oder Butan-1,4-diyl steht
$R^2$ und $R^3$ stehen unabhängig voneinander für eine ($C_1$ bis $C_4$)-Alkylgruppe.

Dabei ist es insbesondere bevorzugt, wenn das obige nichtionische filmbildende und/oder nichtionische festigende Polymer ausgewählt wird aus mindestens einem Polymer, welches mindestens eines oder mehrere der folgenden Merkmale erfüllt:

- $R^1$ bedeutet eine Methylgruppe,
- $X^1$ steht für eine Gruppe NH,
- $A^1$ steht für Ethan-1,2-diyl oder Propan-1,3-diyl,
- $R^2$ und $R^3$, stehen unabhängig voneinander für Methyl oder Ethyl, (besonders bevorzugt für Methyl).

Besonders bevorzugt handelt es sich bei dem nichtionischen filmbildenden und/oder nichtionischen festigenden Polymer dieser Ausführungsform um mindestens ein Polymer, das mindestens eine Struktureinheit der Formel (M-I) und mindestens eine Struktureinheit der Formel (M-III-8) umfasst,

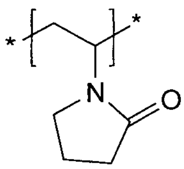 (M-I)

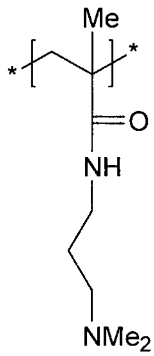 (M-III-8).

Ein ganz besonders bevorzugtes nichtionisches filmbildendes und/oder nichtionisches festigendes Polymer dieser Ausführungsform ist ein Copolymer aus N-Vinylpyrrolidon und N,N-Dimethylaminiopropylmethacrylamid, welches beispielsweise mit der INCI-Bezeichung VP/DMAPA Acrylates Copolymer z.B. unter der Handelsbezeichnung Styleze® CC 10 von der Firma ISP verkauft wird.

Im Rahmen all dieser Ausführungsformen eignen sich die zuvor genannten bevorzugten Ausführungsformen der Polyether-modifizierten organischen Verbindungen als bevorzugt (*vide supra*).

Gleichsam gelten alle zuvor genannten bevorzugten Mengenangaben bezüglich der Komponenten (a) und (b) des erfindungsgemäßen Mittels auch für diese Ausführungsformen *mutatis mutandis* als bevorzugt.

Die erfindungsgemäßen Mittel enthalten die Inhalts- bzw- Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel

Ethanol und Isopropanol enthalten sein.

Es ist erfindungsgemäß bevorzugt mindestens einen ($C_1$ bis $C_4$)-Monoalkylalkohol in den erfindungsgemäßen Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden.

Die erfindungsgemäßen Mittel enthalten vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad \text{(E4-I)}$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$R^4O\text{-}[G]_p$ (E4-II)

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside $C_{12}$-$C_{30}$-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin® CS 15 (COGNIS), Cremophor A25 (BASF SE) bzw. Eumulgin® CS 50 (COGNIS) vermarktet werden.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxygruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel R-O-(CH2-CH2O)x-CH2-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R\text{-}O(CH_2\text{-}CH_2O)_x\text{-}OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

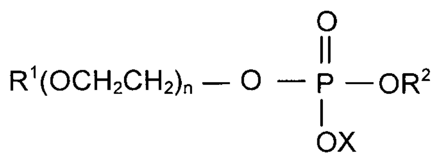

(E1-I)

in der $R^1$ bevorzugt für einen aliphatischen Kohlenwasserstoffrest mit 8 bis 30 Kohlenstoffatomen, $R^2$ für Wasserstoff,

einen Rest $(CH_2CH_2O)_nR^1$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen C1 bis C4 - Kohlenwasserstoffrest, steht,

- sulfatierte Fettsäurealkylenglykolester der Formel (E1-II)

$$R^7CO(AlkO)_nSO_3M \qquad (E1\text{-}II)$$

in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906 beschrieben sind,

- Amidethercarbonsäuren,
- Kondensationsprodukte aus $C_8$ - $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacyl-aminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhy-droxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer $C_8$ - $C_{24}$- Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Al-kylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das $C_{12}$ - $C_{18}$ - Acylsarcosin.

Als weitere geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Das Molgewicht der erfindungsgemäß einsetzbaren Proteinhydrolysate liegt zwischen

75, dem Molgewicht für Glycin, und 200.000, bevorzugt beträgt das Molgewicht 75 bis 50.000 und ganz besonders bevorzugt 75 bis 20.000 Dalton.
Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.
Bevorzugt enthalten die erfindungsgemäßen Mittel Vitamine, Provitamine und Vitaminvorstufen aus den Gruppen A, B, C, E und H.
Panthenol, Pantolacton, Pyridoxin und seine Derivate sowie Nicotinsäureamid und Biotin sind besonders bevorzugt.
Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Als Pflegestoff eignet sich weiterhin eine Reihe von Carbonsäuren.
Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette. Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf die gesamte Anwendungszubereitung, besonders bevorzugt 0,1 bis 5 Gew.%, und insbesondere bevorzugt 0,1 bis 3 Gew.%.

Als Pflegestoff eignen sich weiterhin Ectoin oder Ectoinderivate, Allantoin, Taurin und/oder Bisabolol.

Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Auch Mono- bzw. Oligosaccharide können als Pflegestoff in den erfindungsgemäßen Mitteln eingesetzt werden. Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.
Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.
Erfindungsgemäß geeignete Lipide sind Phospholipide, beispielsweise Sojalecithin, Ei-Lecithin und Kephaline sowie die unter den INCl-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC®

sowie Phospholipid SV® vertrieben.

Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

$$
\begin{array}{l}
CH_2O(CH_2CH_2O)_mR^1 \\
| \\
CHO(CH_2CH_2O)_nR^2 \qquad \text{(D4-I)} \\
| \\
CH_2O(CH_2CH_2O)_qR^3
\end{array}
$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht.

Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt. Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Bevorzugt werden kationische direktziehende Farbstoffe eingesetzt. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Verbindungen die unter den INCI-Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten

sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Mittel können neben den genannten Komponenten weiterhin alle für solche Zubereitungen bekannten Wirk-, Zusatz- und Hilfsstoffe enthalten. Weitere Wirk-, Hilfs- und Zusatzstoffe, sind beispielsweise

**[0137]** - Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate,

- Parfümöle, Dimethylisosorbid und Cyclodextrine,
- Entschäumer wie Silikone,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Konsistenzgeber wie Zuckerester, Polyolester oder Polyolalkylether,
- Fette und Wachse wie Walrat, Bienenwachs, Montanwachs und Paraffine,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die erfindungsgemäßen Mittel als Pumpspray, Aerosolspray, Pumpschaum oder Aerosolschaum.

Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt sind die erfindungsgemäßen Mittel als Aerosolhaarschaum oder Aerosolhaarspray konfektioniert. Das erfindungsgemäße Mittel enthält daher bevorzugt zusätzlich mindestens ein Treibmittel. Erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen. Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis

15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus $N_2O$, Dimethylether, $CO_2$, Luft, Alkanen mit 3 oder 4 Kohlenstoffatomen (wie Propan, n-Butan, iso-Butan) sowie Mischungen daraus.

Die erfindungsgemäßen Mittel und Produkte, die diese Mittel enthalten, insbesondere Aerosolhaarschäume bzw. Aerosolhaarsprays, Gele, zeichnen sich insbesondere dadurch aus, dass sie behandeltem Haar einen sehr starken, dauerhaften Frisurenhalt verleihen, obgleich das Haar bleibt flexibel bleibt.

Ein zweiter Gegenstand der Erfindung ist die Verwendung der erfindungsgemäßen Mittel zur temporären Verformung von Haaren und/oder zur Haarpflege.

Ein dritter Gegenstand der Erfindung ist die Verwendung von Polyether-modifizierten organischen Verbindungen gemäß erstem Erfindungsgegenstand gegen fettige Haut und/oder gegen fettiges Haar (bevorzugt gegen fettiges Haar).

Die Hautfettschmutzabweisung auf der Haut und/oder auf den Haaren (bevorzugt auf den Haaren) wird durch die besagten Polyether gesteigert. Dabei ist es bevorzugt, kosmetische Mittel einzusetzen, die neben mindestens einem der besagten Polyether mindestens ein Tensid enthält.

Es ist ebenso erfindungsgemäß bevorzugt anstelle des Polyethers der Formel (I) mindestens eine Verbindung zu verwenden, die nach dem im ersten Erfindungsgegenstand beschriebenen Herstellverfahren hergestellt wurde. Dabei kann auch ein Gemisch von Reaktionsprodukten aus obigem Herstellverfahren verwendet werden.
Die nach dem ersten Erfindungsgegenstand als bevorzugt gekennzeichneten Polyether eignen sich auch hier bevorzugt. Das gleiche gilt für die im ersten Erfindungsgegenstand erwähnten Tenside. Hierbei ist die Gegenwart mindestens eines anionischen Tensids bevorzugt.

Ein vierter Gegenstand der Erfindung ist die Verwendung des Mittels gemäß erstem Erfindungsgegenstandes zur Verbesserung des Frisurenhaltes.

Die nach dem ersten Erfindungsgegenstand als bevorzugt gekennzeichneten erfindungsgemäßen Mittel eignen sich auch hier bevorzugt.

Die nachfolgenden Beispiele sollen den Gegenstand der vorliegenden Erfindung erläutern ohne ihn in irgendeiner Weise zu beschränken.

**Beispiele**

Die im Beispielteil angegebenen Molekulargewichte sind zahlenmittlere Molekulargewichte der Ausgangsverbindungen (Polyether-Polyole), die zur Herstellung der Präpolymere eingesetzt wurden. Das zahlenmittlere Molekulargewicht der Polyole lässt sich durch Endgruppenbestimmung rechnerisch aufgrund der Kenntnis der Funktionalität der Verbindungen oder der Funktionalität der Mischungskomponenten und der OH-Zahl der Verbindung oder der Mischung (ermittelt nach DIN 53240) bestimmen. Für den Fall, dass als Ausgangsverbindungen andere Verbindungen anstelle der Polyole verwendet werden, ist deren zahlenmittleres Molekulargewicht maßgeblich. So kann beispielsweise das zahlenmittlere Molekulargewicht von Aminen über eine Endgruppenbestimmung durch potentiometrische Titration nach der DIN 16945 ermittelt werden.

Herstellung geeigneter sternförmiger Präpolymere:

<u>Beispiel 1: Dreiarmiger Triethoxysilyl-terminierter Polyether (PP1):</u>

Das verwendete Polyether-Polyol ist ein 3-armiges statistisches Poly(ethylenoxid-co-propylenoxid) mit einem EO/PO-Verhältnis von 75/25 und mit einem zahlenmittleren Molekulargewicht von ca. 5000 g/mol, das von der Firma DOW Chemicals bezogen wurde (Voranol® CP 1421). Vor der Umsetzung wurde das Polyol im Vakuum unter Rühren für 1 h auf 80 °C erwärmt.
Zum getrockneten Polyether-Polyol (2,04 g, 0,41 mmol) wurde (3-Isocyanatopropyl)triethoxysilan (3.17 mg, 1,0 eq.) langsam zugegeben. Die Reaktionsmischung wurde weiter unter Schutzgas bei 100 °C für 2 Tage gerührt, bis die

Schwingungsbande der NCO-Gruppe bei einer IR-Messung verschwunden ist. Man erhält ein Produkt, bei welchem jeweils eine Triethoxysilyl-Gruppe an den freien Enden der Polymerarme des sternförmigen Präpolymers vorhanden ist. Das Produkt ist eine fa rblose, viskose Flüssigkeit.

Beispiel 2: Sechsarmiger Triethoxysilyl-terminierter Polyether (PP2):

Das verwendete Polyether-Polyol ist ein 6-armiges statistisches Poly(ethylenoxid-co-propylenoxid) mit einem EO/PO-Verhältnis von 80/20 und mit einem Molekulargewicht von 12000 g/mol, das durch anionische ringöffnende Polymerisation von Ethylenoxid und Propylenoxid unter Verwendung von Sorbitol als Initiator hergestellt wurde. Vor der Umsetzung wurde das Polyol im Vakuum unter Rühren für 1 h auf 80 °C erwärmt.
Eine Lösung von Polyether-Polyol (3 g, 0,25 mmol), Triethylendiamin (9 mg, 0,081 mmol) und Dibutylzinndilaureat (9 mg, 0,014 mmol) in 25 ml wasserfreiem Toluol wurde vorgelegt, dazu wurde eine Lösung von (3-Isocyanatopropyl)triethoxysilan (0,6 ml, 2,30 mmol) in 10 ml wasserfreiem Toluol tropfenweise zugegeben. Die Lösung wurde weiter bei 50°C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches jeweils eine Triethoxylsilyl-Gruppe an den freien Enden der Polymerarme des sternförmigen Präpolymers aufweist, als eine farblos viskose Flüssigkeit. IR (Film, $cm^{-1}$): 3349 (m, -CO-NH-), 2868 (s, $-CH_2-$,$-CH_3$), 1719 (s, -C=O), 1456 (m, $-CH_2-$, $-CH_3$), 1107 (s, -C-O-C-), 954 (m, -Si-O-). $^1$H-NMR (Benzol-$d_6$, ppm): 1,13 (d, $-CH_3$ von Polymerarmen), 1,21 (t, $-CH_3$ von Silan-Endgruppen), 3,47 (s, $-CH_2$ von Polymerarmen), 3,74 (q, $-CH_2$ von Silan-Endgruppen).

Beispiel 3: Sechsarmiger triethoxysilyl-hydroxy-terminierter Polyether (PP3):

Analog zu Beispiel 2 wurde eine Lösung von Polyether-Polyol (10 g, 0,83 mmol), Triethylendiamin (30 mg, 0,27 mmol) und Dibutylzinndilaureat (30 mg, 0,048 mmol) in 50 ml wasserfreiem Toluol vorgelegt, dazu wurde eine Lösung von (3-Isocyanatopropyl)triethoxysilan (0,65 ml, 2,49 mmol) in 15 ml wasserfreiem Toluol tropfenweise zugegeben. Die Lösung wurde weiter bei 50 °C über Nacht gerührt. Nach dem Entfernen des Toluols unter Vakuum wurde das Rohprodukt durch IR analysiert. Die Ergebnisse zeigten, dass die typischen Schwingungen der NCO-Gruppe bei ca. 2270 $cm^{-1}$ vollständig verschwanden und einhergehend verminderte OH-Schwingungen bei ca. 3351 $cm^{-1}$ zu sehen waren, was darauf hindeutet, dass die Isocyanatosilane-Moleküle über eine Urethan-Bindung erfolgreich an den Enden des Polyols angeknüpft sind. Das Rohprodukt wurde dann wiederholt mit wasserfreiem Ether gewaschen. Nach dem Vakuumtrocknen erhielt man das Produkt, das Triethoxylsilyl- und Hydroxy-Gruppen mit einem statistischen Verhältnis von 3/3 an den freien Enden der Polymerarme der sternförmigen Präpolymere aufweist, als eine farblos viskose Flüssigkeit. IR (Film, $cm^{-1}$): 3511, (m, -OH), 3351 (m, -CO-NH-), 2868 (s, $-CH_2-$, $-CH_3$), 1720 (s, -C=O), 1456 (m, $-CH_2-$, $-CH_3$), 1112 (s, -C-O-C-), 953 (m, -Si-O-). $^1$H-NMR (Benzol-$d_6$, ppm): 1,08-1,17 (m, $-CH_3$ von Polymerarmen und $-CH_3$ von Silan-Endgruppen), 3,47 (s, $-CH_2$ von Polymerarmen), 3,74 (q, $-CH_2$ von Silan-Endgruppen).

Beispiel 4: Sechsarmiger triethoxysilyl-hydroxy-terminierter Polyether (PP4):

Analog zu Beispiel 2 wurde eine Lösung von Polyether-Polyol (10 g, 0,83 mmol), Triethylendiamin (30 mg, 0,27 mmol) und Dibutylzinndilaureat (30 mg, 0,048 mmol) in 50 ml wasserfreiem Toluol vorgelegt. Dazu wurde eine Lösung von (3-Isocyanatopropyl)triethoxysilan (0,22 ml, 0,84 mmol) in 15 ml wasserfreiem Toluol tropfenweise gegeben. Die Lösung wurde weiter bei 50 °C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches Triethoxylsilyl- und Hydroxy-Gruppen mit einem statistischen Verhältnis von 1/5 an den freien Enden der Polymerarme der sternförmigen Präpolymere aufweist, als eine farblos viskose Flüssigkeit. IR (Film, $cm^{-1}$): 3494, (m, -OH), 3346 (w, -CO-NH-), 2868 (s, $-CH_2-$, $-CH_3$), 1722 (m,-C=O), 1456 (m, $-CH_2-$, $-CH_3$), 1112 (s, -C-O-C-), 952 (m, -Si-O-). $^1$H-NMR (Benzol-$d_6$, ppm): 1,08-1,18 (m, $-CH_3$ von Polymerarmen und $-CH_3$ von Silan-Endgruppen), 3,49 (s, $-CH_2$ von Polymerarmen), 3,75 (q, $-CH_2$ von Silan-Endgruppen).

Beispiel 5: Sechsarmiger Triethoxysilyl-terminierter Polyether (PP5):

Das verwendete Polyether-Polyol ist ein 6-armiges statistisches Poly(ethylenoxid-co-propylenoxid) mit einem EO/PO-Verhältnis von ca. 80/20 und mit einem zahlenmittleren Molekulargewicht von ca. 3000 g/mol, das durch anionische ringöffnende Polymerisation von Ethylenoxid und Propylenoxid unter Verwendung von Sorbitol als Initiator hergestellt wurde. Vor der Umsetzung wurde das Polyether-Polyol im Vakuum unter Rühren für 1 h auf 80 °C erwärmt.

Zu dem getrockneten Polyether-Polyol (20 g, 6,67 mmol) wurde Dibutylzinndilaureat (2 mg, 0,01%) und (3-Isocyanatopropyl)triethoxysilan (9,5 g, 1,0 eq.) langsam zugegeben. Die Reaktionsmischung wurde weiter unter Schutzgas bei Raumtemperatur für 2 Tage gerührt, bis die NCO-Band bei IR-Messung verschwunden ist. Man erhielt ein

Produkt, welches jeweils eine Triethoxylsilyl-Gruppe an den freien Enden der Polymerarme der sternförmigen Präpolymere aufweist, als eine farblos viskose Flüssigkeit.

Beispiel 6: Mischung aus dreiarmigem Triethoxysilyl-terminiertem Polyether und achtarmigem Triethoxysilyl-terminiertem Polyether (PP6):

Die verwendete Polyether-Polyol-Mischung besteht aus einem 3-armigen statistischen Poly(ethylenoxid-co-propylenoxid) (Glycerin-gestartet) und einem 8-armigen Polyether-Polyol (Rohrzucker-gestartet). Die Polymerarme sind jeweils statistische Poly(ethylenoxid-co-propylenoxide) mit einem EO/PO-Verhältnis von 75/25. Die OH-Funktionalität beträgt durchschnittlich 6,9 (durch Endgruppenbestimmung ermittelt), wodurch sich ein durchschnittliches zahlenmittleres Molekulargewicht von ca. 12000 g/mol ergibt. Es ergibt sich ein Verhältnis von 78 Gew.-% an 8-armigem Polyether-Polyol zu 22 Gew.-% an 3-armigem Polyether-Polyol. Das Polyether-Polyol-Gemisch wurde von der Firma DOW Chemicals bezogen (Voranol® 4053). Vor der Umsetzung wurde das Polyether-Polyol im Vakuum unter Rühren für 1 h auf 80 °C erwärmt.

Zum getrockneten Polyether-Polyol (209 g, 16,9 mmol) wurde Dibutylzinndilaureat (20,9 mg, 0,01 %) und (3-Isocyanatopropyl)triethoxysilan (30,3 g, 1,0 eq.) langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme der beiden sternförmigen Präpolymere vorhanden ist. Das Produkt ist eine farblose, viskose Flüssigkeit.

Messung des statischen Wasserkontaktwinkels und der Kontaktwinkelhysterese

Die Messungen wurden mit einem Kontaktwinkelmessgerät der Firma Data Physics GmbH durchgeführt (Gerätetyp: OCA20; elektronische Kippvorrichtung TBU90E; elektronisches Spritzenmodul ES; Software: SCA inkl. Software-Update für SCA-Module (Version 3.11.6 Build 155)).

Das Gerät wird vor der Messung mittels der Kalibrierungsautomatik des Geräts kalibriert. Mittels des Spritzenmoduls wird ein Tropfen destilliertes Wasser (15 $\mu$l) auf die zu messende Oberfläche des Objektträgers aufgebracht. Der Kippwinkel beträgt 0°, d.h. die zu messende Fläche ist waagerecht. Es erfolgt eine Aufnahme des Tropfens mit einer Videokamera. Am Einzelbild wird mittels der Software eine Tangente am Tropfenquerschnitt an dem Punkt angelegt, an dem der Tropfen die Oberfläche berührt. Der sich ergebende Winkel zwischen der Tangente und der zu messenden Oberfläche wird als statischer Kontaktwinkel bezeichnet (Sessile-Drop-Verfahren).

Anschließend wird die Probe samt Probentisch und der Kamera bei der langsamsten Geschwindigkeit, die das Gerät zulässt (rechnerisch aus den Geräteangaben: 0,62 °/s) bis zu einem Winkel von 90° gekippt. Während dieses Vorgangs wird ein Video des Tropfens durch die Kamera mittels der Software aufgenommen, wobei der Kippwinkel zum Zeitpunkt der Aufnahme mitgespeichert wird. Sobald der Tropfen beginnt von der Oberfläche abzulaufen wird die Messung beendet. Mittels der Software kann anschließend im Video der Fortschreitwinkel (Winkel in Tropfenfließrichtung) und der Rückzugswinkel (auf der anderen Seite des Tropfens) mittels der Ellipsen-Methode der Messsoftware zum Zeitpunkt des beginnenden Ablaufens des Tropfens von der Oberfläche bestimmt werden. Die Differenz der beiden Winkel ist die Kontaktwinkelhysterese (Tilting-Plate-Verfahren).

Bereitstellung von Haarbehandlungsmitteln

Folgende Haarbehandlungsmittel wurden durch Vermischen der Inhaltsstoffe hergestellt:

Tabelle 1: Haargel

| Rohstoff | Gew.-% |
|---|---|
| Benzophenone-4 | 0,05 |
| Synthalen K | 1,0 |
| Neolone PE | 0,6 |
| PP1 | 1,0 |
| PVPNA Copolymer 60/40 W | 5,0 |
| Luviskol K 85 CQ Loesung | 4,0 |
| Dow Corninq 193 Fluid | 0,2 |

(fortgesetzt)

| Rohstoff | Gew.-% |
|---|---|
| Neutrol TE | 1,3 |
| Antara 430 | 0,1 |
| D-Panthenol | 0,1 |
| PEG-40 Hydrogenated Castor Oil | 0,5 |
| Parfum | 0,2 |
| Wasser | ad 100 |

Tabelle 2: Haargel

| Rohstoff | Gew.-% |
|---|---|
| Benzophenone-4 | 0,05 |
| Synthalen K | 0,5 |
| Solan ELD | 0,1 |
| Methylparaben | 0,1 |
| Dekafald | 0,1 |
| 1,2-Propandiol | 0,1 |
| PVPNA Copolymer 60/40 W | 10,0 |
| PP3 | 0,5 |
| N,N,N',N'-Ethylendiamintetraacetat Dinatriumsalz | 0,01 |
| Sorbitol | 1,0 |
| D-Panthenol | 0,1 |
| Luviset Clear | 0,2 |
| Triethanolamin | 0,3 |
| Luviskol K 85 CQ Loesung | 5,0 |
| PEG-40 Hydrogenated Castor Oil | 0,4 |
| Parfum | 0,1 |
| Wasser | ad 100 |

Tabelle 3: Haargel

| Rohstoff | Gew.-% |
|---|---|
| Dow Corning 9045 | 8,0 |
| Dow Corning 1501 Fluid | 8,0 |
| Simulgel EG | 2,0 |
| PP5 | 0,7 |
| 1,2-Propandiol | 1,7 |
| D-Panthenol | 0,2 |
| Herbasol Destillat Seerose | 0,5 |
| 2-Phenoxyethanol | 0,5 |

(fortgesetzt)

| Rohstoff | Gew.-% |
|---|---|
| Dekafald | 0,1 |
| Luviskol K 85 CQ Loesung | 10,0 |
| Solubilisant LRI | 0,6 |
| Parfum | 0,1 |
| Wasser | ad 100 |

Tabelle 4: Haarspray (nicht erfindungsgemäß)

| Rohstoff | Gew.-% |
|---|---|
| Ethanol vergällt mit n-Butanol/Bitrex® | ad 100 |
| AMP Ultra PC 1000 | 0,825 |
| Amphomer | 3,0 |
| PP1 | 0,3 |
| p-Methoxyzimtsäureisoamylester | 0,1 |
| Triethylcitrat | 0,1 |
| Isopropylmyristat | 0,05 |
| Parfum | 0,25 |
| Isobutan | 30 |

Tabelle 5: Haarschaum

| Rohstoff | Gew.-% |
|---|---|
| Hydagen HCMF | 0,3 |
| Milchsäure | 0,1 |
| Luviquat FC 370 | 1,0 |
| Celquat L-200 | 0,5 |
| Luviskol K 85 CQ | 2,0 |
| PP1 | 2,0 |
| Natriumbenzoat | 0,3 |
| Benzophenone-4 | 0,1 |
| D-Panthenol | 0,1 |
| Glycerin | 0,2 |
| Genamin CTAC | 1,022 |
| PEG-40 Hydrogenated Castor Oil | 0,306 |
| Parfum | 0,102 |
| Propan/Butan | 6 |
| Wasser | ad 100 |

Tabelle 6: Schampoo (nicht erfindungsgemäß)

| Rohstoff | Gew.-% |
|---|---|
| Texapon N 70 | 15,4 |
| PP3 | 4,0 |
| Natriumhydroxid | 0,14 |
| Zitronensäure Monohvdrat | 0,5 |
| Salicylsäure | 0,2 |
| Disodium Cocoamphodiacetate | 7,0 |
| Arlypon F | 0,5 |
| Natriumbenzoat | 0,5 |
| Euperlan PK 3000 AM | 2,6 |
| D-Panthenol | 0,1 |
| Nikotinsäureamid | 0,1 |
| Cutina HR | 0,1 |
| Cetiol HE | 0,8 |
| Polymer JR 400 | 0,3 |
| Co-Enzym Q 10 | 0,01 |
| Parfum | 0,1 |
| Natriumchlorid | 0,5 |
| Wasser | ad 100 |

Curl Retention

Es wurden folgende Zusammensetzungen bereitgestellt:

| | | |
|---|---|---|
| A: | Polyvinylpyrrolidon | 5 Gew.-% |
| | Wasser | 95 Gew.-% |
| | | |
| B: | Polyvinylpyrrolidon | 5 Gew.-% |
| | PP6 | 1 Gew.-% |
| | Wasser | 94 Gew.-% |

Es wurden standardisierte Haarsträhnen der Fa. Kerling (Art. Nr. 827560) des Haartyps "European Natural, Farbe 6/0) von einer Länge ($L_{max}$) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet. 0.18g der Zusammensetzungen wurden auf jeweils eine Haarsträhne appliziert und einmassiert. Die Strähne wurde sodann auf einen Wickler gewickelt (Fripac-medis, ∅ 7mm, Art. Nr. D-1203) und über Nacht bei Raumtemperatur getrocknet.
Die Wickler wurden vorsichtig entfernt und die Strähne aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85% über einen Zeitraum von 24 h gelagert und danach die Längen der Locken erneut vermessen (Lt).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{max} - L_t}{L_{max} - L_0}$$

| | |
|---|---|
| HHCR A: | 38 % |
| HHCR B: | 43 % |

Der Zusatz der erfindungsgemäßen Polyether modifizierten organischen Verbindungen bewirkt eine Verbesserung der Curl Retention bei hoher Luftfeuchtigkeit.

**Patentansprüche**

**1.** Kosmetisches Mittel, enthaltend in einem kosmetisch akzeptablen Träger

(a) Polyether modifizierte, organische Verbindungen, welche mindestens drei Polyether-Substituenden aufweisen, wobei die Polyether eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette,

und

(b) mindestens ein nichtionisches filmbildendes und/oder nichtionisches festigendes Polymer.

**2.** Kosmetisches Mittel nach Anspruch 1, enthaltend in einem kosmetisch akzeptablen Träger

(a) Polyether-modifizierte organische Verbindungen, welche ausgewählt werden aus mindestens einer Verbindung der allgemeinen Formel (PE-1)

$$[Q] \!-\! (K'\text{-}A\text{-}K\text{-}T)_n \qquad \text{(PE-1)}$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus

anionischem Rest,

Rest $-Si(OR)_x(R')_{3-x}$

worin R und R' unabhängig voneinander für eine ($C_1$ bis $C_4$)- Alkylgruppe (insbesondere für Methyl oder Ethyl) stehen,
Q ein organisches Strukturfragment, abgeleitet von linearen, verzweigten, zyklischen oder heterozyklischen Kohlenwasserstoffen, wobei all diese jeweils gesättigt, ungesättigt oder aromatisch sein dürfen,
n für eine ganze Zahl von 3 bis 64 steht

und
(b) mindestens ein filmbildendes und/oder festigendes Polymer.

**3.** Kosmetisches Mittel nach Anspruch 1 oder 2, enthaltend in einem kosmetisch akzeptablen Träger

(a) Polyether-modifizierte organische Verbindungen, welche durch Umsetzung von

(i) organischen Verbindungen enthaltend mindestens drei Reste, ausgewählt aus Hydroxygruppe und/oder Aminogruppe mit
(ii) mindestens 3 Moläquivalenten mindestens eines Polyethers der Formel (I) erhalten werden

T-K-A-K'-Y (I)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T für ein Molekülfragment steht, umfassend mindestens einen Substituenten ausgewählt aus

anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen, x für 1, 2 oder 3 steht
Y für eine gegenüber eine Hydroxygruppe oder Aminogruppe reaktive Gruppe steht,

und
(b) mindestens ein filmbildendes und/oder festigendes Polymer.

**4.** Mittel nach Anspruch 1 oder 2, enthaltend in einem kosmetisch akzeptablen Träger

(a) Polyether-modifizierte organische Verbindungen, welche durch Umsetzung von

(i) organischen Polyether-Polyol-Verbindungen umfassend

- mindestens drei Polyoxyalkylenketten aus Ethylenoxideinheiten oder aus Ethylenoxid- und Propylenoxid-Einheiten, mit einem maximalen Anteil von 50 Gew.-% an Propylenoxid-Einheiten, bezogen auf das Gewicht der Polyoxyalkylenkette,
- mindestens drei Hydroxygruppen

mit
(ii) mindestens 3 Moläquivalenten mindestens einer Verbindung der Formel (III)

Y-K-T (III)

worin,

Y für eine gegenüber -OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$

worin R und R' unabhängig voneinander für eine ($C_1$ bis $C_4$)-Alkylgruppe (insbesondere für Methyl oder Ethyl) stehen,
x für 1, 2 oder 3 steht.

und
(b) mindestens ein filmbildendes und/oder festigendes Polymer.

**5.** Mittel nach einem der Ansprüche 1 bis 4, **dadurch gekennzeichnet, dass** die besagten Polyether-modifizierten organischen Verbindungen in einer Menge von 0,01 bis 10,0 Gew.-% bezogen auf das Gewicht des gesamten Mittels enthalten sind.

**6.** Mittel nach einem der Ansprüche 1 bis 5, **dadurch gekennzeichnet, dass** A steht für ein Strukturfragment der Formel (A1)

$$*\text{-}(OCH_2CH_2)_n\text{-}(OCH_2CH(CH_3))_m\text{-}* \qquad (A1)$$

worin

n eine ganze Zahl von 1 bis 500 bedeutet,
m eine ganze Zahl von 0 bis 500 bedeutet und
das Strukturfragment der Formel (A1) einen maximalen Anteil von 50 Gew.-% an Propylenoxid-Einheiten, bezogen auf das Gewicht des Strukturfragments (A1) hat.

**7.** Mittel nach einem der Ansprüche 1 bis 6, **dadurch gekennzeichnet, dass** die Reste K beziehungsweise K' unabhängig voneinander für eine kovalente Bindung, eine Oxygruppe, eine Iminogruppe, eine ($C_1$ bis $C_6$)-Alkylengruppe oder mindestens eine der folgenden Konnektivitäten (K1) bis (K10) stehen

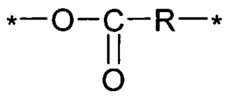
(K1)

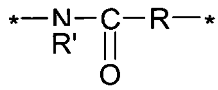
(K2)

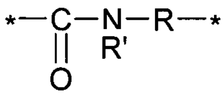
(K4)

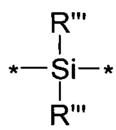
(K5)

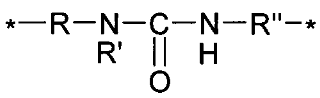
(K7)

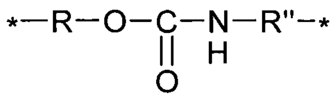
(K8)

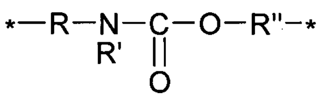
(K9)

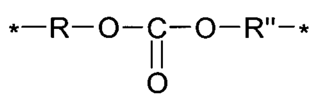
(K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,
R' ein Wasserstoffatom oder eine ($C_1$ bis $C_4$)-Alkylgruppe bedeutet,
R''' unabhängig voneinander eine ($C_1$ bis $C_4$)-Alkylgruppe oder eine Arylgruppe bedeutet.

**8.** Mittel nach einem der Ansprüche 1 bis 7, **dadurch gekennzeichnet, dass** die Polyether modifizierten organischen Verbindungen ausgewählt werden aus mindestens einer Verbindung der Formel (PE-1a) oder (PE-1b) oder (PE-1c) oder (PE-1d) oder (PE-1e) oder (PE-1f) oder Mischungen daraus,

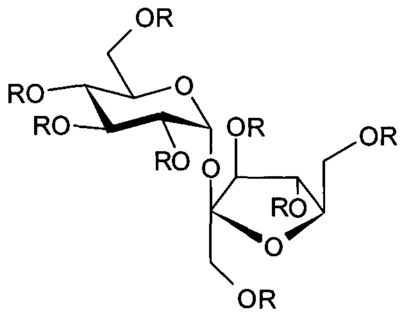

(PE-1a)

worin

mindestens drei Gruppen R für eine Gruppe $-(CH_2CH_2O)_p-(CHCH_3CH_2O)_m-K-T$ stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

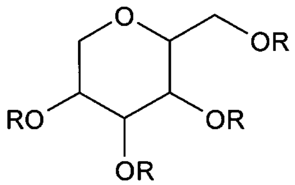

(PE-1b)

worin
mindestens drei Gruppen R für eine Gruppe $-(CH_2CH_2O)_p-(CHCH_3CH_2O)_m-K-T$ stehen und die übrigen Gruppen R für ein Wasserstoffatom oder eine Gruppe -K-T stehen, worin unabhängig voneinander
p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

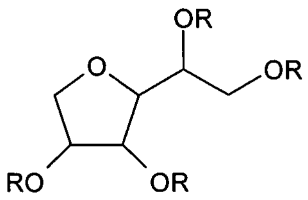

(PE-1c)

worin

mindestens drei Gruppen R für eine Gruppe $-(CH_2CH_2O)_p-(CHCH_3CH_2O)_m-K-T$ stehen und die übrigen Gruppen R für ein Wasserstoffatom oder eine Gruppe -K-T stehen, worin unabhängig voneinander p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

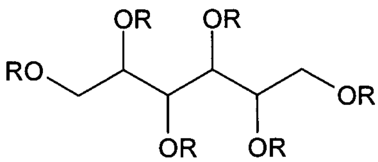

(PE-1d)

worin

mindestens drei Gruppen R für eine Gruppe $-(CH_2CH_2O)_p-(CHCH_3CH_2O)_m-K-T$ stehen und die übrigen Gruppen R für ein Wasserstoffatom oder eine Gruppe -K-T stehen, worin unabhängig voneinander p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht;

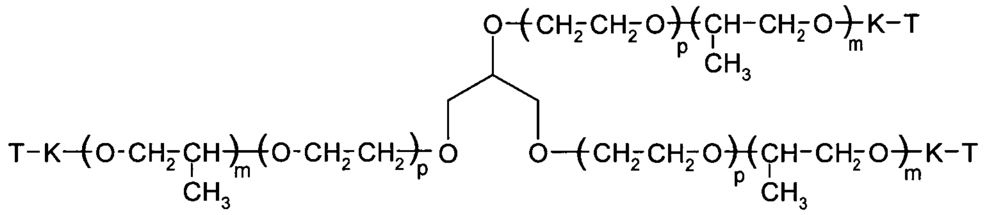

(PE-1f)

worin unabhängig voneinander
p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,

K steht für eine Konnektivität, ausgewählt aus einer kovalenten Bindung oder aus einem Molekülfragment mit zwei freien Valenzen,
T steht für ein Molekülfragment umfassend mindestens einen Substituenten ausgewählt aus anionischem Rest

Rest $-Si(OR)_x(R')_{3-x}$,

worin R und R' unabhängig voneinander für eine ($C_1$ bis $C_4$)-Alkylgruppe (insbesondere für Methyl oder Ethyl) stehen, x für 1, 2 oder 3 steht.

**9.** Mittel nach einem der Ansprüche 1 bis 8, **dadurch gekennzeichnet, dass** das Molekülfragment T der allgemeinen Formel

$$*-B-[(N(CH_2COOM)-B')_y-N(CH_2COOM)_2]$$

folgt, wobei

B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,
M unabhängig voneinander für ein Wasserstoffatom oder ein Äquivalent eines ein- oder mehrwertigen Kations,
y 1 oder 2 (vorzugsweise 1) bedeutet.

**10.** Mittel nach einem der Ansprüche 1 bis 8, **dadurch gekennzeichnet, dass** T für einen Rest-$Si(OR)_x(R')_{3-x}$ steht, worin R und R' unabhängig voneinander für eine ($C_1$ bis $C_4$)-Alkylgruppe (insbesondere für Methyl oder Ethyl) stehen und x für 2 oder 3 steht.

**11.** Mittel nach einem der Ansprüche 1 bis 10, **dadurch gekennzeichnet, dass** die filmbildenden und/oder festigenden Polymere in einer Menge von 0,1 Gew.-% bis 20,0 Gew.-% bezogen auf das Gewicht des Mittels enthalten sind.

**12.** Verwendung eines Mittels nach einem der Ansprüche 1 bis 11 zur temporären Verformung von Haaren.

**13.** Verwendung eines Mittels nach einem der Ansprüche 1 bis 11 zur Verbesserung des Frisurenhaltes.

## Claims

**1.** A cosmetic agent containing, in a cosmetically acceptable carrier,

(a) polyether-modified organic compounds comprising at least three polyether substituents, wherein the polyethers comprise a polyoxyalkylene chain consisting of ethylene oxide units or consisting of ethylene oxide units and propylene oxide units, containing a maximum of 50 % by weight of propylene oxide units, based on the

weight of the polyoxyalkylene chain,

and

(b) at least one non-ionic film-forming and/or non-ionic setting polymer.

**2.** The cosmetic agent according to claim 1, containing, in a cosmetically acceptable carrier,

(a) polyether-modified organic compounds selected from at least one compound of general formula (PE-1)

$$[Q]\!-\!(K'\text{-}A\text{-}K\text{-}T)_n \qquad \text{(PE-1)}$$

where

A denotes a polyoxyalkylene chain consisting of ethylene oxide units or consisting of ethylene oxide units and propylene oxide units, containing a maximum of 50 % by weight of propylene oxide units, based on the weight of A,
K and K' represent, independently of each other, a connectivity selected from a covalent bond or from a molecular fragment having two free valencies,
T represents a molecular fragment comprising at least one substituent selected from
an anionic functional group, -

$$Si(OR)_x(R')_{3-x}$$

functional group, where R and R' represent, independently of each other, a ($C_1$ to $C_4$) alkyl group (in particular methyl or ethyl),
Q is an organic structural fragment taken from linear, branched, cyclic or heterocyclic hydrocarbons, wherein each of these hydrocarbons may be saturated, unsaturated or aromatic,
n represents an integer from 3 to 64

and

(b) at least one film-forming and/or setting polymer.

**3.** The cosmetic agent according to one of claims 1 or 2, containing, in a cosmetically acceptable carrier,

(a) polyether-modified organic compounds which are obtained by reacting

(i) organic compounds containing at least three functional groups selected from hydroxyl groups and/or amino groups with
(ii) at least three mol equivalents at least of a polyether of formula (I)

$$T\text{-}K\text{-}A\text{-}K'\text{-}Y \qquad \text{(I)}$$

where

A denotes a polyoxyalkylene chain consisting of ethylene oxide units or consisting of ethylene oxide units and propylene oxide units, containing a maximum of 50% by weight of propylene oxide units, based on the weight of A,
K and K' represent, independently of each other, a connectivity selected from a covalent bond or from a molecular fragment having two free valencies,
T represents a molecular fragment comprising at least one substituent selected from
an anionic functional group an

$$-Si(OR)_x(R')_{3-x}$$

functional group,

where R and R' represent, independently of each other, a ($C_1$ to $C_4$) alkyl group (in particular methyl or ethyl), and
x represents 1, 2 or 3,

Y represents a group that is reactive to a hydroxyl group or amino group,

and

(b) at least one film-forming and/or setting polymer.

**4.** The agent according to one of claims 1 or 2, containing, in a cosmetically acceptable carrier,

(a) polyether-modified organic compounds which are obtained by reacting

(i) organic polyether polyol compounds comprising

- at least three polyoxyalkylene chains consisting of ethylene oxide units or consisting of ethylene oxide units and propylene oxide units, containing a maximum of 50 % by weight of propylene oxide units, based on the weight of the polyoxyalkylene chain, and
- at least three hydroxyl groups

with
(ii) at least three mol equivalents at least of a compound of formula (III)

$$Y\text{-}K\text{-}T \qquad (III)$$

where

Y represents a group that is reactive to -OH, $-NH_2$, -NHR, $-NR_2$,
K represents a connectivity selected from a covalent bond or from a molecular fragment having two free valencies,
T represents a molecular fragment comprising at least one substituent selected from an anionic functional group, an

$$-Si(OR)_x(R')_{3-x}$$

functional group,

where R and R' represent, independently of each other, a ($C_1$ to $C_4$) alkyl group (in particular methyl or ethyl), and
x represents 1, 2 or 3,

and
(b) at least one film-forming and/or setting polymer.

**5.** The agent according to one of claims 1 to 4, **characterized in that** said polyether-modified organic compounds are contained in an amount of 0.01 to 10.0 % by weight, based on the weight of the total agent.

**6.** The agent according to one of claims 1 to 5, **characterized in that** A represents a structural fragment of formula (A1)

$$*\text{-}(OCH_2CH_2)_n\text{-}(OCH_2CH(CH_3))_m\text{-}* \qquad (A1)$$

where

n denotes an integer from 1 to 500,
m denotes an integer from 0 to 500, and

the structural fragment of formula (A1) contains a maximum of 50 % by weight of propylene oxide units, based on the weight of the structural fragment (A1).

**7.** The agent according to any of claims 1 to 6, **characterized in that** the functional groups K and K' represent, independently of each other, a covalent bond, an oxy group, an imino group, a ($C_1$ to $C_6$) alkylene group or at least one of the following connectivities (K1) to (K10),

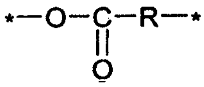
(K1)

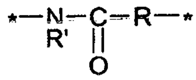
(K2)

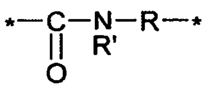
(K4)

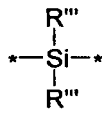
(K5)

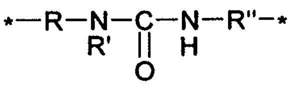
(K7)

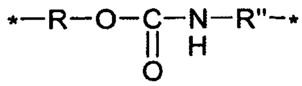
(K8)

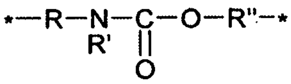
(K9)

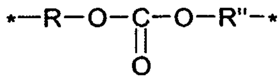
(K10)

where

R and R" represent, independently of each other, methylene, ethane-1,2-diyl, propane-1,2-diyl, propane-1,3-diyl, butane-1,2-diyl, butane-1,3-diyl, butane-1,4-diyl or phenylene,
R' is a hydrogen atom or a ($C_1$ to $C_4$) alkyl group,
R"' independently denotes a ($C_1$ to $C_4$) alkyl group or an aryl group.

**8.** The agent according to one of claims 1 to 7, **characterized in that** the polyether-modified organic compounds are selected from at least one compound of formula (PE-1a) or (PE-1b) or (PE-1c) or (PE-1d) or (PE-1e) or (PE-1f) or mixtures thereof,

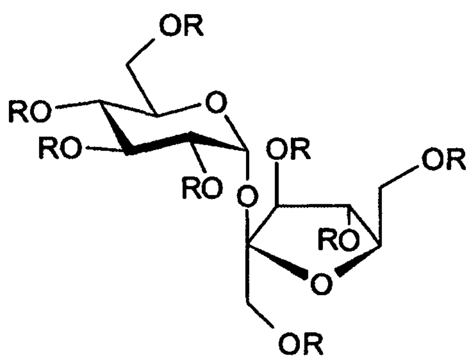
(PE-1a)

where
at least three R groups represent a $-(CH_2CH_2O)_p-(CHCH_3CH_2O)_m-K-T$ group and the remaining R groups represent a hydrogen atom or a -K-T group, where, independently of one another,

p denotes an integer from 1 to 500 and m denotes an integer from 0 to 500, and p and m are in a ratio to one

another such that the maximum content of propylene oxide units is 50 % by weight (preferably a maximum of 40 % by weight, and particularly preferably a maximum of 30 % by weight), based on the weight of the corresponding polyoxyalkylene chains,

K represents a connectivity selected from a covalent bond or from a molecular fragment having two free valencies,
T represents a molecular fragment comprising at least one substituent selected from an anionic functional group an $-Si(OR)_x(R')_{3-x}$ functional group, where R and R' represent, independently of each other, a ($C_1$ to $C_4$) alkyl group (in particular methyl or ethyl), and x represents 1, 2 or 3;

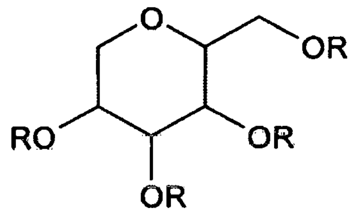

(PE-1b)

where
at least three R groups represent a $-(CH_2CH_2O)_p-(CHCH_3CH_2O)_m-K-T$ group and the remaining R groups represent a hydrogen atom or a -K-T group, where, independently of one another,
p denotes an integer from 1 to 500 and m denotes an integer from 0 to 500, and p and m are in a ratio to one another such that the maximum content of propylene oxide units is 50 % by weight (preferably a maximum of 40 % by weight, and particularly preferably a maximum of 30 % by weight), based on the weight of the corresponding polyoxyalkylene chains,

K represents a connectivity selected from a covalent bond or from a molecular fragment having two free valencies,
T represents a molecular fragment comprising at least one substituent selected from an anionic functional group an $-Si(OR)_x(R')_{3-x}$ functional group, where R and R' represent, independently of each other, a ($C_1$ to $C_4$) alkyl group (in particular methyl or ethyl), and x represents 1, 2 or 3;

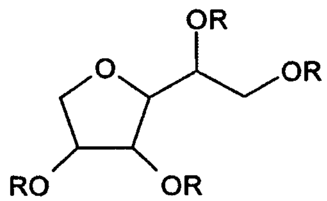

(PE-1c)

where
at least three R groups represent a $-(CH_2CH_2O)_p-(CHCH_3CH_2O)_m-K-T$ group and the remaining R groups represent a hydrogen atom or a -K-T group, where, independently of one another,
p denotes an integer from 1 to 500 and m denotes an integer from 0 to 500, and p and m are in a ratio to one another such that the maximum content of propylene oxide units is 50 % by weight (preferably a maximum of 40 % by weight, and particularly preferably a maximum of 30 % by weight), based on the weight of the corresponding polyoxyalkylene chains,

K represents a connectivity selected from a covalent bond or from a molecular fragment having two free valencies,
T represents a molecular fragment comprising at least one substituent selected from
an anionic functional group
an $-Si(OR)_x(R')_{3-x}$ functional group,
where R and R' represent, independently of each other, a ($C_1$ to $C_4$) alkyl group (In particular methyl or ethyl), and x represents 1, 2 or 3;

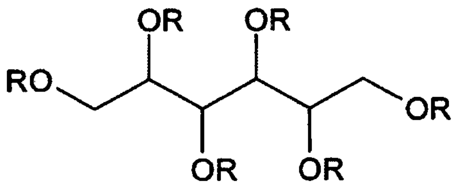

(PE-1d)

where

at least three R groups represent a $-(CH_2CH_2O)_p-(CHCH_3CH_2O)_m-K-T$ group and the remaining R groups represent a hydrogen atom or a -K-T group, where, independently of one another,

p denotes an integer from 1 to 500 and m denotes an integer from 0 to 500, and p and m are in a ratio to one another such that the maximum content of propylene oxide units is 50 % by weight (preferably a maximum of 40 % by weight, and particularly preferably a maximum of 30 % by weight), based on the weight of the corresponding polyoxyalkylene chains,

K represents a connectivity selected from a covalent bond or from a molecular fragment having two free valencies,

T represents a molecular fragment comprising at least one substituent selected from an anionic functional group an $-Si(OR)_x(R')_{3-x}$ functional group, where R and R' represent, independently of each other, a ($C_1$ to $C_4$) alkyl group (in particular methyl or ethyl), and x represents 1, 2 or 3;

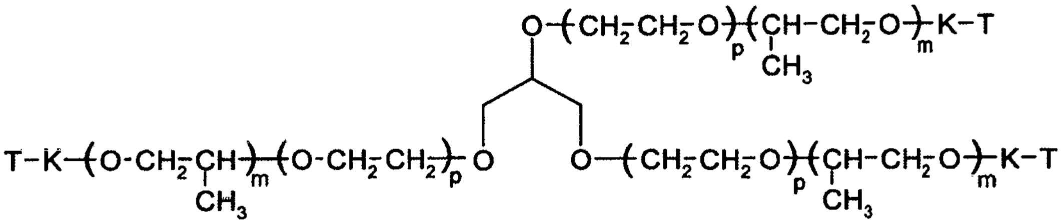

(PE-1f)

where, independently of one another,

p denotes an integer from 1 to 500 and m denotes an integer from 0 to 500, and p and m are in a ratio to one another such that the maximum content of propylene oxide units is 50 % by weight (preferably a maximum of 40 % by weight, and particularly preferably a maximum of 30 % by weight), based on the weight of the corresponding polyoxyalkylene chains,

K represents a connectivity selected from a covalent bond or from a molecular fragment having two free valencies,

T represents a molecular fragment comprising at least one substituent selected from an anionic functional group an $-Si(OR)_x(R')_{3-x}$ functional group, where R and R' represent, independently of each other, a ($C_1$ to $C_4$) alkyl group (in particular methyl or ethyl), and x represents 1, 2 or 3.

9. The agent according to one of claims 1 to 8, **characterized in that** the molecular fragment T has the general formula

$$*\text{-B-[(N(CH}_2\text{COOM)-B)}_y\text{-N(CH}_2\text{COOM)}_2]$$

where

B represents a ($C_1$ to $C_6$) alkylene functional group (preferably ethane-1,2-diyl, propane-1,2-diyl, propane-1,3-diyl, butane-1,2-diyl, butane-1,3-diyl, butane-1,4-diyl),

B' represents a ($C_1$ to $C_6$) alkylene functional group (preferably ethane-1,2-diyl, propane-1,2-diyl, propane-1,3-diyl, butane-1,2-diyl, butane-1,3-diyl, butane-1,4-diyl) or an N,N-bis($C_1$ to $C_6$)-alkylene-N-carboxymethyl,

M independently represents a hydrogen atom or an equivalent of a monovalent or multivalent cation,

y denotes 1 or 2 (preferably 1).

10. The agent according to one of claims 1 to 8, **characterized in that** T represents a - $Si(OR)_x(R')_{3-x}$functional group,

where R and R' represent, independently of each other, a ($C_1$ to $C_4$) alkyl group (in particular methyl or ethyl) and x represents 2 or 3.

**11.** The agent according to one of claims 1 to 10, **characterized in that** the film-forming and/or setting polymers are contained in an amount of from 0.1 % by weight to 20.0 % by weight, based on the weight of the agent.

**12.** The use of an agent according to one of claims 1 to 11 for temporarily styling hair.

**13.** The use of an agent according to one of claims 1 to 11 for improving the hold of a hairstyle.

**Revendications**

**1.** Agent cosmétique comprenant dans un support cosmétiquement acceptable,

(a) des composés organiques, modifiés par des polyéthers, qui possèdent au moins trois substituants polyéther, les polyéthers comprenant une chaîne polyoxyalkylène constituée d'unités oxyde d'éthylène ou d'unité oxyde d'éthylène et oxyde de propylène, avec une proportion maximale de 50% en poids d'unités oxyde de propylène, sur la base du poids de la chaîne polyoxyalkylène,

et

(b) au moins un polymère filmogène non-ionique et/ou un polymère de renforcement non-ionique.

**2.** Agent cosmétique selon la revendication 1, contenant dans un support cosmétiquement acceptable

(a) des composés organiques, modifiés par des polyéthers, qui sont choisis parmi au moins un composé de la formule générale (PE-1)

$$[Q]\text{-}(K'\text{-}A\text{-}K\text{-}T)_n \qquad \text{(PE-1)}$$

dans laquelle

A est une chaîne polyoxyalkylène oxyde constituée d'unités oxyde d'éthylène ou d'unités oxyde d'éthylène ou oxyde de propylène, avec une proportion maximale de 50% en poids d'unités oxyde de propylène, par rapport au poids de A,
K et K' représentent indépendamment une connectivité choisie parmi une liaison covalente ou un fragment de molécule ayant deux valences libres,
T représente un fragment moléculaire comprenant au moins un substituant choisi parmi
un radical anionique le radical

$$-Si(OR)_x(R')_{3-x}$$

où R et R' représentent indépendamment un groupe alkyle (en particulier méthyle ou éthyle) en $C_1$ à $C_4$,
Q représente un fragment structurel organique dérivé de substances hydrocarbonées linéaires, ramifiées, cycliques ou hétérocycliques, toutes ces substances pouvant être saturées, insaturés ou aromatiques,
n représente un nombre entier allant de 3 à 64

et

(b) au moins un polymère filmogène et/ou un polymère de renforcement.

**3.** Agent cosmétique selon la revendication 1 ou 2, contenant dans un support cosmétiquement acceptable

(a) des composés organiques, modifiés par des polyéthers, qui sont obtenus par réaction

(i) de composés organiques contenant au moins trois radicaux choisis parmi un groupe hydroxy et/ou un groupe amino avec
(ii) au moins 3 équivalents molaires d'au moins un polyéther de la formule (I)

T-K-A-K'-Y (I)

où

A est un groupe polyoxyalkylène constitué d'unités oxyde d'éthylène ou d'unités oxyde d'éthylène et oxyde de propylène, avec une proportion maximale de 50% en poids d'unités oxyde de propylène, par rapport au poids de A,
K et K' représentent choisis indépendamment une connexion choisie parmi une liaison covalente ou un fragment de molécule ayant deux valences libres,
T représente un fragment de molécule comprenant au moins un substituant choisi parmi un radical anionique le radical

$-Si(OR)_x(R')_{3-x}$

où R et R' représentent indépendamment un groupe alkyle (en particulier méthyle ou éthyle) en $C_1$ à $C_4$, x représentent 1, 2 ou 3
Y représente un groupe réactif vis-à-vis d'un groupe hydroxy ou d'un groupe amino,

et

(b) au moins un polymère filmogène et/ou un polymère de renforcement.

**4.** Agent selon la revendication 1 ou 2, contenant dans un support cosmétiquement acceptable

(a) des composés organiques, modifiés par des polyéthers, qui sont obtenus par réaction

(i) de composés organiques polyéther-polyol comprenant

- au moins trois chaînes polyoxyalkylène constituées d'unités oxyde d'éthylène ou d'unités oxyde d'éthylène et oxyde de propylène, avec une proportion maximale de 50% en poids d'unités oxyde de propylène, par rapport au poids de la chaîne polyoxyalkylène,
- au moins trois groupes hydroxy,

avec
(ii) au moins 3 équivalents molaires d'au moins un composé de la formule (III)

Y-K-T (III)

dans laquelle,

Y représente un groupe réactif vis-à-vis de -OH, $-NH_2$, -NHR, $-NR_2$,
K représente une connectivité choisie parmi une liaison covalente ou un fragment de molécule ayant deux valences libres,
T représente Un fragment de molécule comprenant au moins un substituant choisi parmi un radical anionique le radical $-Si(OR)_x(R')_{3-x}$ où R et R' représentent indépendamment un groupe alkyle (en particulier méthyle ou éthyle) en $C_1$ à $C_4$, x représentent 1, 2 ou 3

et
(b) au moins un polymère filmogène et/ou un polymère de renforcement.

**5.** Agent selon l'une des revendications 1 à 4, **caractérisé en ce que** lesdits composés organiques, modifiés par des polyéthers, sont contenus dans une quantité allant de 0,01 à 10,0% en poids sur la base du poids de tout l'agent.

**6.** Agent selon l'une des revendications 1 à 5, **caractérisé en ce que** A représente un fragment structurel de la formule

(A1),

$$*\text{-}(OCH_2CH_2)_n\text{-}(OCH_2CH(CH_3))_m\text{-}* \qquad (A1)$$

dans laquelle

n est un nombre entier de 1 à 500,
m est un nombre entier de 0 à 500, et
le fragment structurel de la formule (A1) présente une proportion maximale de 50% en poids d'unités oxyde de propylène, par rapport au poids du fragment structurel (A1).

**7.** Agent selon l'une des revendications 1 à 6, **caractérisé en ce que** les radicaux K ou K' représentent indépendamment une liaison covalente, un groupe oxy, un groupe imino, un groupe alkylène en $C_1$ à $C_6$, ou au moins une des connectivités (K1) (K10) suivantes

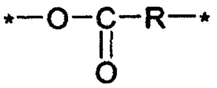
(K1)

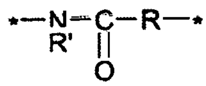
(K2)

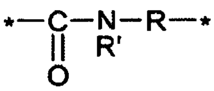
(K4)

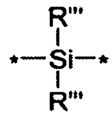
(K5)

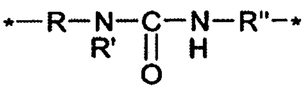
(K7)

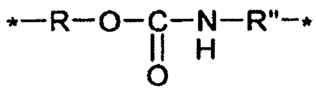
(K8)

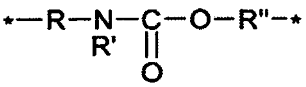
(K9)

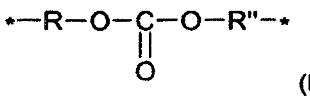
(K10)

dans lesquelles

R et R" représentent indépendamment un groupe méthylène, éthane-1,2-diyle, propane-1,2-diyle, propane-1,3-diyle, butane-1,2-diyle, butane-1,3-diyle, butane-1,4-diyle ou phénylène,
R' représente un atome d'hydrogène ou un groupe alkyle en $C_1$ à $C_4$,
R'" représentent indépendamment un groupe alkyle en $C_1$ à $C_4$ ou un groupe aryle.

**8.** Agent selon l'une des revendications 1 à 7, **caractérisé en ce que** les composés organiques, modifiés par des polyéthers, sont choisis parmi au moins un composé de la formule (PE-1a) ou (PE-1b) ou (PE-1c) ou (PE-1d) ou (PE-1e) ou (PE-1f), ou leurs mélanges,

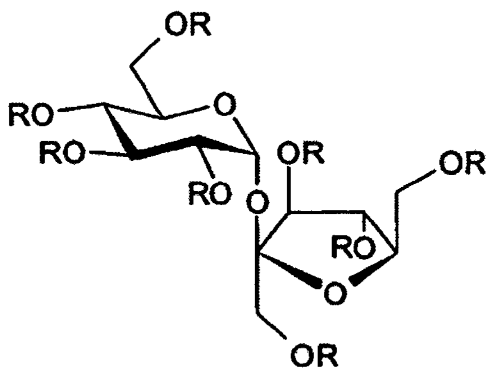

(PE-1a)

dans laquelle

au moins trois groupes R représente un groupe $-(CH_2CH_2O)_p-(CHCH_3CH_2O)_m-K-T$ et les groupes restants R représentent un atome d'hydrogène ou un groupe -K-T, où indépendamment

p est un nombre entier de 1 à 500 et m est un nombre entier de 0 à 500, et p et m sont dans un rapport entre eux avec une proportion maximale de 50% en poids (de préférence maximale de 40% en poids, plus préférablement maximale de 30% en poids) d'unités oxyde de propylène, par rapport au poids de la chaîne polyoxyalkylène correspondante,

K représente une connectivité choisie parmi une liaison covalente ou un fragment de molécule ayant deux valences libres,
T est un fragment de molécule comprenant au moins un substituant choisi parmi un radical anionique le radical $-Si(OR)_x(R')_{3-x}$ où R et R' représentent indépendamment un groupe alkyle (en particulier méthyle ou éthyle) en $C_1$ à $C_4$, x représentent 1, 2 ou 3 ;

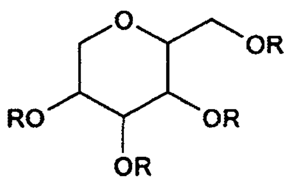

(PE-1b)

où

au moins trois groupes R représentent un groupe $-(CH_2CH_2O)_p-(CHCH_3CH_2O)_m-K-T$ et les groupes restants R représentent un atome d'hydrogène ou un groupe -K-T, où indépendamment
p est un nombre entier de 1 à 500 et m est un nombre entier de 0 à 500, et p et m sont dans un rapport entre eux avec une proportion maximale de 50% en poids (de préférence maximale de 40% en poids, plus préférablement maximale de 30% en poids) d'unités oxyde de propylène, par rapport au poids de la chaîne polyoxyalkylène correspondante,

K représente une connectivité choisie parmi une liaison covalente ou un fragment de molécule ayant deux valences libres,
T est un fragment de molécule comprenant au moins un substituant choisi parmi un radical anionique le radical $-Si(OR)_x(R')_{3-x}$ où R et R' représentent indépendamment un groupe alkyle (en particulier méthyle ou éthyle) en $C_1$ à $C_4$, x représentent 1, 2 ou 3 ;

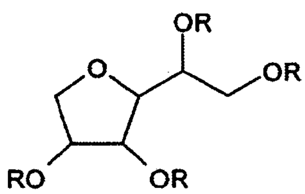

(PE-1c)

où

au moins trois groupes R représentent un groupe $-(CH_2CH_2O)_p-(CHCH_3CH_2O)_m-K-T$ et les groupes restants R représentent un atome d'hydrogène ou un groupe -K-T, où indépendamment

p est un nombre entier de 1 à 500 et m est un nombre entier de 0 à 500, et p et m sont dans un rapport entre eux avec une proportion maximale de 50% en poids (de préférence maximale de 40% en poids, plus préférablement maximale de 30% en poids) d'unités oxyde de propylène, par rapport au poids de la chaîne polyoxyalkylène correspondante,

K représente une connectivité choisie parmi une liaison covalente ou un fragment de molécule ayant deux valences libres,

T est un fragment de molécule comprenant au moins un substituant choisi parmi un radical anionique le radical $-Si(OR)_x(R')_{3-x}$ où R et R' représentent indépendamment un groupe alkyle (en particulier méthyle ou éthyle) en $C_1$ à $C_4$, x représentent 1, 2 ou 3 ;

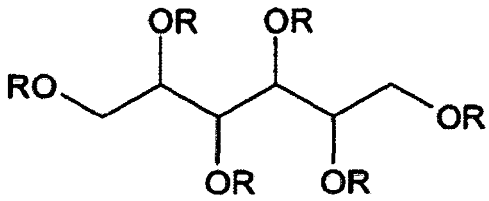

(PE-1d)

où

au moins trois groupes R représentent un groupe $-(CH_2CH_2O)_p-$ $(CHCH_3CH_2O)_m-K-T$ et les groupes restants R représentent un atome d'hydrogène ou un groupe -K-T, indépendamment

p est un nombre entier de 1 à 500 et m est un nombre entier de 0 à 500, et p et m sont dans un rapport entre eux avec une proportion maximale de 50% en poids (de préférence maximale de 40% en poids, plus préférablement maximale de 30% en poids) d'unités oxyde de propylène, par rapport au poids de la chaîne polyoxyalkylène correspondante,

K représente une connectivité choisie parmi une liaison covalente ou un fragment de molécule ayant deux valences libres,

T est un fragment de molécule comprenant au moins un substituant choisi parmi un radical anionique le radical

$$-Si(OR)_x(R')_{3-x}$$

où R et R' représentent indépendamment un groupe alkyle (en particulier méthyle ou éthyle) en $C_1$ à $C_4$, x représentent 1, 2 ou 3 ;

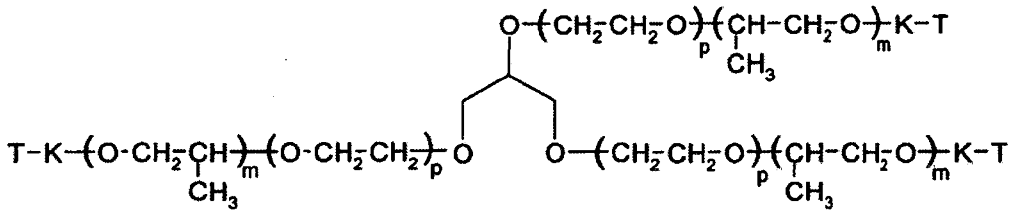

(PE-1f)

où indépendamment

p est un nombre entier de 1 à 500 et m est un nombre entier de 0 à 500, et p et m sont dans un rapport entre eux avec une proportion maximale de 50% en poids (de préférence maximale de 40% en poids, plus préférablement maximale de 30% en poids) d'unités oxyde de propylène, par rapport au poids de la chaîne polyoxyalkylène correspondante,

K représente une connectivité choisie parmi une liaison covalente ou un fragment de molécule ayant deux valences libres,

T est un fragment de molécule comprenant au moins un substituant choisi parmi un radical anionique le radical $-Si(OR)_x(R')_{3-x}$ où R et R' représentent indépendamment un groupe alkyle (en particulier méthyle ou éthyle) en $C_1$ à $C_4$, x représentent 1, 2 ou 3.

**9.** Agent selon l'une des revendications 1 à 8, **caractérisé en ce que** le fragment de molécule T a la formule générale

$$*-B-[(N(CH_2COOM)-B')_y-N(CH_2COOM)_2],$$

dans laquelle

B représente un radical alkylène en $C_1$ à $C_6$, (de préférence un groupe éthane-1,2-diyle, propane-1,2-diyle, propane-1,3-diyle, butane-1,2-diyle, butane-1,3-diyle, butane-1,4-diyle),
B' représente un radical alkylène en $C_1$ à $C_6$ (de préférence un groupe éthane-1,2-diyle, propane-1,2-diyle, propane-1,3-diyle, butane-1,2-diyle, butane-1,3-diyle, butane-1,4-diyle) ou N,N-bis-alkylène en $C_1$ à $C_6$-N-carboxyméthyle,
M représente indépendamment un atome d'hydrogène ou un équivalent d'un cation monovalent ou multivalent,
y est égal à 1 ou 2 (de préférence 1).

**10.** Agent selon l'une des revendications 1 à 8, **caractérisé en ce que** T représente un radical $-Si(OR)_x(R')_{3-x}$, dans lequel R et R' représentent indépendamment un groupe alkyle en $C_1$ à $C_4$ (en particulier un méthyle ou un éthyle), et x est égal à 2 ou 3.

**11.** Agent selon l'une des revendications 1 à 10, **caractérisé en ce que** les polymères filmogènes et/ou les polymères de renforcement sont présents dans une quantité allant de 0,1% en poids à 20,0% en poids par rapport au poids de l'agent.

**12.** Utilisation d'un agent selon l'une des revendications 1 à 12 pour la mise en forme temporaire des cheveux.

**13.** Utilisation d'un agent selon l'une des revendications 1 à 12 pour améliorer la tenue de la coiffure.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009024450 A2
- WO 2007096056 A1
- EP 1226813 A2
- DE OS19738866 A
- DE OS19736906 A
- DE OS19756454 A
- EP 998908 A2